(19) Europäisches Patentamt
European Patent Office
Office européen des brevets

(11) **EP 4 203 782 B1**

(12) **EUROPEAN PATENT SPECIFICATION**

(45) Date of publication and mention
of the grant of the patent:
**12.06.2024 Bulletin 2024/24**

(21) Application number: **20775747.7**

(22) Date of filing: **28.08.2020**

(51) International Patent Classification (IPC):
**A61B 5/08** *(2006.01)*    **A61B 5/0507** *(2021.01)*
**A61B 5/00** *(2006.01)*    **G01S 13/88** *(2006.01)*
**A61B 5/11** *(2006.01)*    **G05B 15/02** *(2006.01)*
**H04L 12/28** *(2006.01)*

(52) Cooperative Patent Classification (CPC):
**A61B 5/0816; A61B 5/0022; A61B 5/0507;**
**A61B 5/1118; A61B 5/4815; A61B 5/4818;**
**A61B 5/6898; A61B 5/7267; A61B 5/741;**
**A61B 5/742; G01S 7/415; G01S 7/417;**
**G01S 13/343; G01S 13/886; H04L 12/2823;**

(Cont.)

(86) International application number:
**PCT/US2020/048388**

(87) International publication number:
**WO 2022/046072 (03.03.2022 Gazette 2022/09)**

(54) **CONTACTLESS DEVICE FOR RESPIRATORY HEALTH MONITORING**

KONTAKTLOSE VORRICHTUNG ZUR ÜBERWACHUNG DER ATEMGESUNDHEIT

DISPOSITIF SANS CONTACT POUR SURVEILLANCE DE LA SANTÉ RESPIRATOIRE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**05.07.2023 Bulletin 2023/27**

(73) Proprietor: **Google LLC**
**Mountain View, CA 94043 (US)**

(72) Inventors:
• **SHIN, Dongeek**
**Mountain View, California 94043 (US)**
• **SCHNEIDER, Logan**
**Mountain View, California 94043 (US)**
• **GOLDENSON, Andrew William**
**Mountain View, California 94043 (US)**

(74) Representative: **Maikowski & Ninnemann**
**Patentanwälte Partnerschaft mbB**
**Postfach 15 09 20**
**10671 Berlin (DE)**

(56) References cited:
**WO-A1-2019/122413    WO-A2-2020/104465**
**AU-A1- 2012 308 234    US-A1- 2011 060 215**

• **FAN YANG ET AL: "EM techniques for the**
**detection of breast cancer", ANTENNAS AND**
**PROPAGATION SOCIETY INTERNATIONAL**
**SYMPOSIUM (APSURSI), 2010 IEEE, IEEE,**
**PISCATAWAY, NJ, USA, 11 July 2010**
**(2010-07-11), pages 1-4, XP032146298, DOI:**
**10.1109/APS.2010.5562289 ISBN:**
**978-1-4244-4967-5**

EP 4 203 782 B1

(52) Cooperative Patent Classification (CPC): (Cont.)
A61B 5/01; A61B 2505/07

**Description**

CROSS-REFERENCES TO RELATED APPLICATIONS

This application is related to PCT Application US2019/031,290, filed May 8, 2019, entitled "Sleep Tracking and Vital Sign Monitoring Using Low Power Radio Waves." This application is also related to Non-Provisional Application No. 16/990,705, filed on August 11, 2020, entitled "Contactless Sleep Detection and Disturbance Attribution" (Attorney Docket No.: 090421-1183285). This application is also related to Non-Provisional Application No. 16/990,714, filed on August 11, 2020, entitled "Contactless Sleep Detection and Disturbance Attribution for Multiple Users" (Attorney Docket No.: 090421-1183289). This application is also related to Non-Provisional Application No. 16/990,720, filed on August 11, 2020, entitled "Contactless Cough Detection and Attribution" (Attorney Docket No.: 090421-1183290). This application is also related to Non-Provisional Application No. 16/990,726, filed on August 11, 2020, entitled "Precision Sleep Tracking Using a Contactless Sleep Tracking Device" (Attorney Docket No.: 090421-1190042). This application is also related to Non-Provisional Application No. 16/990,746, filed on August 11, 2020, entitled "Initializing Sleep Tracking on a Contactless Health Tracking Device" (Attorney Docket No.: 090421-1198051).

BACKGROUND

When persons are sleeping, they may experience health issues that they are unaware of, especially if they tend to sleep alone. While a person may tend to experience symptoms such as waking up frequently, experiencing fatigue, excessive sleepiness, or poor exercise performance, the person may not identify these symptoms or may not attribute these symptoms to a medical issue that presents itself when the person is asleep. For example, paradoxical breathing, which can be related to sleep apnea, can result in a person experiencing some or all of these noted symptoms. Such a person may benefit from having attributes of their health, such as breathing, monitored while sleeping. Contactless health monitoring devices are disclosed in AU 2012 308 234 A1 and WO 2019/122413 A1. WO 2020/104465 A2 discloses a method and an apparatus for detection of disordered breathing. US 2011/060215 A1 discloses an apparatus and a method for non-invasive and continuous measurement of respiratory chamber volume and associated parameters. FAN YANG ET AL: "EM techniques for the detection of breast cancer", ANTENNAS AND PROPAGATION SOCIETY INTERNATIONAL SYMPOSIUM (APSURSI), 2010 IEEE, IEEE, PISCATAWAY, NJ USA, 11 July 2010 (2010-07-11), pages 1 -4, XP032146298, DOI: 10.1109/APS.2010.5562289, ISBN: 978-1-4244-4967-5 describes the using of radar based microwave imaging technique for breast cancer detection.

SUMMARY

The proposed solution relates to a contactless health monitoring device as stated in claim 1 of the accompanying claim set. Various embodiments are described related to a contactless health monitoring device. In some embodiments, a contactless health monitoring device is described. The device may comprise a housing. The device may comprise a radar sensor that may comprise a plurality of antennas. The radar sensor may be housed by the housing. The device may comprise a processing system, comprising one or more processors, in communication with the radar sensor, housed by the housing. The processing system may be configured to receive, for each antenna of the plurality of antennas, a radar data stream from the radar sensor, thereby receiving a plurality of radar data streams. The processing system may be configured to perform a beam steering process that may create a plurality of targeted spatial zone radar data streams from the received plurality of radar data streams. The processing system may be configured to determine breathing displacement for a user for each targeted spatial zone radar data stream of the plurality of targeted spatial zone radar data streams. The processing system may be configured to analyze the breathing displacement for the user for each targeted spatial zone radar data stream of the plurality of targeted spatial zone radar data streams. The processing system may be configured to output a screening result based on analyzing the breathing displacement for the user in relation to time for each targeted spatial zone radar data stream of the plurality of targeted spatial zone radar data streams.
Embodiments of such a device may include one or more of the following features: the screening result may indicate the user may be experiencing paradoxical breathing. The beam steering process may comprise applying weighted delay and sum (WDAS) beam steering to create the plurality of targeted spatial zone radar data streams. The processing system being configured to determine the breathing displacement for the user for each targeted spatial zone radar data stream of the plurality of targeted spatial zone radar data steams may comprise the processing system being configured to determine a phase value for each targeted spatial zone radar data stream of the plurality of targeted spatial zone radar data streams. The processing system being configured to analyze the breathing displacement for the user for each targeted spatial zone radar data stream of the plurality of targeted spatial zone radar data streams may comprise the processing system being configured to: apply a pre-trained machine learning model. The pre-trained machine learning model may be a neural network. Each targeted spatial zone of the plurality of targeted spatial zones may correspond to a different portion of user's body. The plurality of targeted spatial zones may comprise at least five targeted spatial zones. The processing system may be further configured to: determine, using a state machine, that the user is in a sleep state and not moving based on the plurality of radar data streams. The device may further comprise a wireless network interface, housed by the housing and in communication with the processing system. The device may further comprise a touchscreen display, housed by the housing, and in communication with the processing system. The device may further comprise a microphone housed by the housing and in communication with the processing system. The device may further comprise a speaker housed by the housing and in communication with the processing system. The processing system may be further configured to receive a spoken command regarding analyzing the breathing displacement for the user. The processing system may be further configured to cause the spoken request to be transmitted to a cloud-based server system via the wireless network interface. The processing system may be further configured to receive a command via the wireless network interface in response to transmitting the spoken request to the cloud-based server system. The processing system may be further configured to output the screening result via the touchscreen display at least partially based on the received command.

Furthermore, a method for performing contactless respiratory health monitoring is described. The method may comprise receiving, for each antenna of a plurality of antennas of a radar subsystem, a radar data stream, thereby receiving a plurality of radar data streams. The method may comprise performing, by one or more processors, a beam steering process that may create a plurality of targeted spatial zone radar data streams from the received plurality of radar data streams. The method may comprise determining, by the one or more processors, breathing displacement for a user for each targeted spatial zone radar data stream of the plurality of targeted spatial zone radar data streams. The method may comprise analyzing, by the one or more processors, the breathing displacement for the user for each targeted spatial zone radar data stream of the plurality of targeted spatial zone radar data streams. The method may comprise outputting, by the one or more processors, a screening result based on analyzing the breathing displacement for the user in relation to time for each targeted spatial zone radar data stream of the plurality of targeted spatial zone radar data streams.

Such a method may also include one or more of the following features: the screening result may indicate the user may be experiencing paradoxical breathing. The beam steering process may comprise applying weighted delay and sum (WDAS) beam steering to create the plurality of targeted spatial zone radar data streams. Determining the breathing displacement for the user for each targeted spatial zone radar data stream of the plurality of targeted spatial zone radar data steams may comprise determining a phase value for each targeted spatial zone radar data stream of the plurality of targeted spatial zone radar data streams. Analyzing the breathing displacement for the user for each targeted spatial zone radar data stream of the plurality of targeted spatial zone radar data streams may comprise applying a pre-trained machine learning model. The pre-trained machine learning model may be a neural network. Each targeted spatial zone of the plurality of targeted spatial zones may correspond to a different portion of user's body. The plurality of targeted spatial zones may comprise at least three targeted spatial zones. The method may further comprise: determining, using a state machine, that the user is in a sleep state and not moving based on the plurality of radar data streams.

In some embodiments, a contactless health monitoring device is presented. The device can include a housing. The device can include a radar sensor that comprises a plurality of antennas, wherein the radar sensor is housed by the housing. The device can include a processing system, comprising one or more processors, in communication with the radar sensor, housed by the housing. The processing system can be configured to receive, for each antenna of the plurality of antennas, a radar data stream from the radar sensor, thereby receiving a plurality of radar data streams. The processing system can be configured to perform a beam steering process that creates a plurality of targeted spatial zone radar data streams from the received plurality of radar data streams. The processing system can be configured to determine breathing displacement for a user for each targeted spatial zone radar data stream of the plurality of targeted spatial zone radar data streams. The processing system can be configured to analyze the breathing displacement for the user for each targeted spatial zone radar data stream of the plurality of targeted spatial zone radar data streams. The processing system can be configured to output a screening result based on analyzing the breathing displacement for the user in relation to time for each targeted spatial zone radar data stream of the plurality of targeted spatial zone radar data streams. A first of said targeted spatial zone radar streams may correspond to a chest zone of the user. A second of the targeted spatial zone radar streams can correspond to an abdomen zone of the user. The analyzing can include detecting a phase difference between the breathing displacement of the abdomen zone and the breathing displacement of the chest zone sufficient to be indicative of a paradoxical breathing condition. The output screening result can include information representative of the detected paradoxical breathing condition.

BRIEF DESCRIPTION OF THE DRAWINGS

A further understanding of the nature and advantages of various embodiments may be realized by reference to the following figures. In the appended figures, similar components or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.

**FIG. 1** illustrates a diagram defining paradoxical breathing.

**FIG. 2** illustrates an example of a user's breathing being monitored while sleeping.

**FIG. 3** illustrates a block diagram of an embodiment of a contactless health monitoring system.

**FIG. 4A** illustrates an embodiment of a contactless health monitoring system.

**FIG. 4B** illustrates an embodiment of frequency-modulated continuous wave radar radio waves output by a radar subsystem.

**FIG. 5A** illustrates an embodiment of a phase capture module that may be used as part of a contactless health monitoring system.

**FIG. 5B** illustrates an embodiment of a contactless health monitoring system that monitors multiple spatial zones.

**FIG. 6** illustrates an embodiment of a beam steering module for a contactless sleep tracking device that targets the direction in which sleep tracking is performed.

**FIG. 7** illustrates an embodiment of the antenna layout of a radar subsystem that may be used in combination with a beam steering module of a contactless sleep tracking device.

**FIG. 8** illustrates another embodiment of a beam steering module for targeting the direction in which sleep tracking is performed.

**FIG. 9** illustrates an embodiment of a health tracking system that uses radar to detect human interactions and perform health monitoring with integrated beam steering.

**FIG. 10** illustrates an exploded view of an embodiment of a contactless health tracking device.

**FIG. 11** illustrates an embodiment of a state system for determining when a person is sleeping.

**FIG. 12** illustrates an embodiment of a method for performing contactless monitoring of a user's breathing.

DETAILED DESCRIPTION

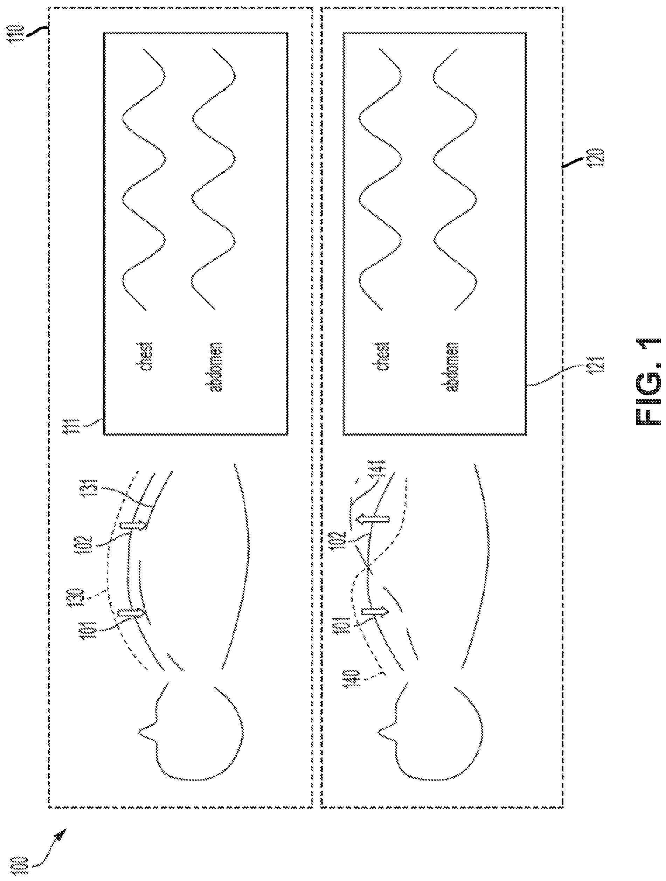

In paradoxical breathing, a person's chest movement and diaphragm (or, more generally, abdomen) movement are out of synchronization. **FIG. 1** illustrates diagram 100 defining paradoxical breathing. Normal breathing example 110 illustrates that a person's chest 101 and abdomen 102 tend to move in approximate synchronization with each other, as indicated by expanded chest/abdomen 130 and contracted chest/abdomen 131. That is, when the person breathes, chest 101 and abdomen 102 tend to move outward and inward at approximately the same time. In example 111, chest 101 and abdomen 102 move in and out in synchronization, resulting in a constructive summed movement waveform when individual waveforms representing the movement of the person's chest and person's abdomen are summed.

Paradoxical breathing example 120 involves chest 101 moving outward (expanding) when abdomen 102 is moving inward and chest 101 moving inward when abdomen 102 is moving outward, as represented by chest expanded/abdomen contracted state 140 and chest contracted/abdomen expanded state 141. This condition results in the person having less air enter and exit the person's lungs, thus decreasing the amount of oxygen present in the person's lungs and, therefore, the oxygenation level of the person's blood. Symptoms a person may experience from paradoxical breathing can include: waking up frequently, fatigue, excessive sleepiness (when awake), and/or poor athletic performance. Further, paradoxical breathing might be symptomatic of a larger health issue, such as sleep apnea. In paradoxical breathing, when waveforms, as indicated in example 121, indicative of movement of chest 101 and abdomen 102 are summed, complete or significant destructive interference is present. Additionally or alternatively, embodiments detailed herein can aid in identifying respiratory effort and patency of the airway, therefore allowing for the estimation of the presence of and differentiation between breathing issues of central and neurologic (e.g., brain, brainstem, spine), physiologic (e.g., genetic/chemostatic, heart failure, altitude, narcotics, etc.), intrinsic (e.g., nerve, muscle diseases), and extrinsic origin (e.g., obstructive or restrictive pulmonary disorders).

Embodiments detailed herein are focused on a contactless health monitoring device that can determine that paradoxical breathing, or some other breathing condition, has occurred and can provide the user with an indication of the condition, possibly along with a recommendation to seek professional medical advice. Such contactless monitoring can occur while the user is sleeping. The contactless health monitoring device may be installed bedside and, once set up, may determine when a user is sleeping and may monitor the user for paradoxical breathing and/or other breathing conditions that can be detected based on movement of the user's chest and/or abdomen. The contactless health monitoring device uses radar to monitor various portions of the user's chest and/or abdomen. (For simplicity, the remainder of this document refers exclusively to the user's chest; however, it should be understood that the term chest can be interpreted to include at least a portion of the user's abdomen.) The movement occurring across different parts of the user's chest can be used to determine if paradoxical breathing is occurring. Other breathing conditions may be monitored using similar systems and methods to those detailed herein. For example, shallow breathing and irregular breathing may be identified using similar systems and methods.

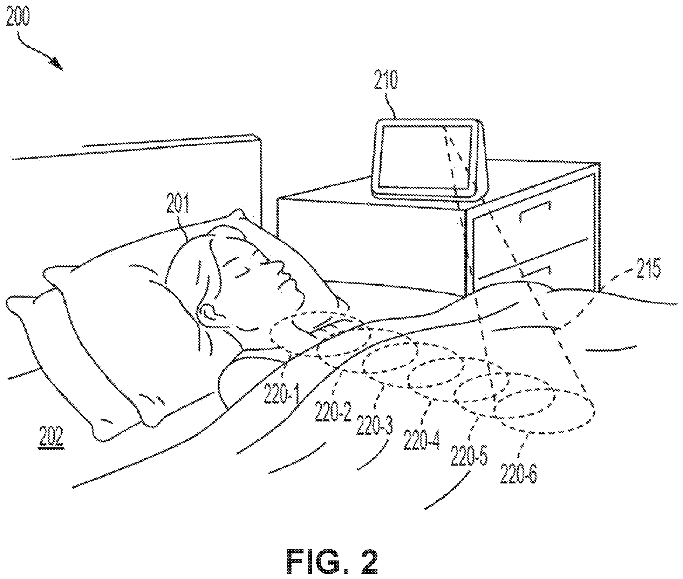

**FIG. 2** illustrates embodiment 200 of a user's breathing being monitored while sleeping using embodiments detailed herein. In embodiment 200, user 201 is sleeping in bed 202. Contactless health monitoring device 210 ("device 210") is located bedside, such as on a nightstand. Radar may be used to detect movement of user 201 attributed to breathing. Sheets, blankets, clothing, and other inanimate objects may be penetrated by the radio waves, which may be millimeter waves, output by contactless health monitoring device 210. Therefore, breathing of user 201 may be monitored despite blankets, sheets, and clothing being in the direct path between contactless health monitoring device 210 and the chest of user 201.

Spatial zones 220 may be monitored by device 210. Each of spatial zones 220 may correspond to a different region of the chest of user 201. Spatial zones 220 can partially overlap. In other embodiments, spatial zones 220 are spaced farther apart using beam steering to increase, decrease, or eliminate overlap. In some embodiments, two adjacent spatial zones overlap by less than 50%. The number of spatial zones 220 may vary by embodiment. In some embodiments, as few as two spatial zones may be present. In some embodiments, 3, 4, 5, or more spatial zones 220 are present. In still other embodiments, 6 spatial zones may be present, such as illustrated in embodiment 200.

Each of spatial zones 220 (e.g., spatial zones 220-1, 220-2, 220-3, 220-4, 220-5, and 220-6) may be individually targeted using beam steering. By device 210 having multiple antennas, beam steering may be performed using processing performed by device 210. Therefore, a single radar chirp of radio waves may be emitted and reflections may be received using multiple antennas of device 210. Data obtained from the received reflected radio waves may have a beam steering process applied to effectively target a particular spatial zone of spatial zones 220. This same data may have the beam steering process applied multiple times (e.g., using different weights), thereby allowing targeting of each of spatial zones 220 using the same dataset. For example, various weights may be applied to create beam form 215 to target spatial zone 220-6. A separate set of weights may be applied to the same data to perform beam steering and target spatial region 220-5. Because the same data set based on reflected radio waves is processed using multiple beam steering processes, movement occurring in one of spatial zones 220 can be simultaneously and separately monitored from movement in other spatial zones 220.

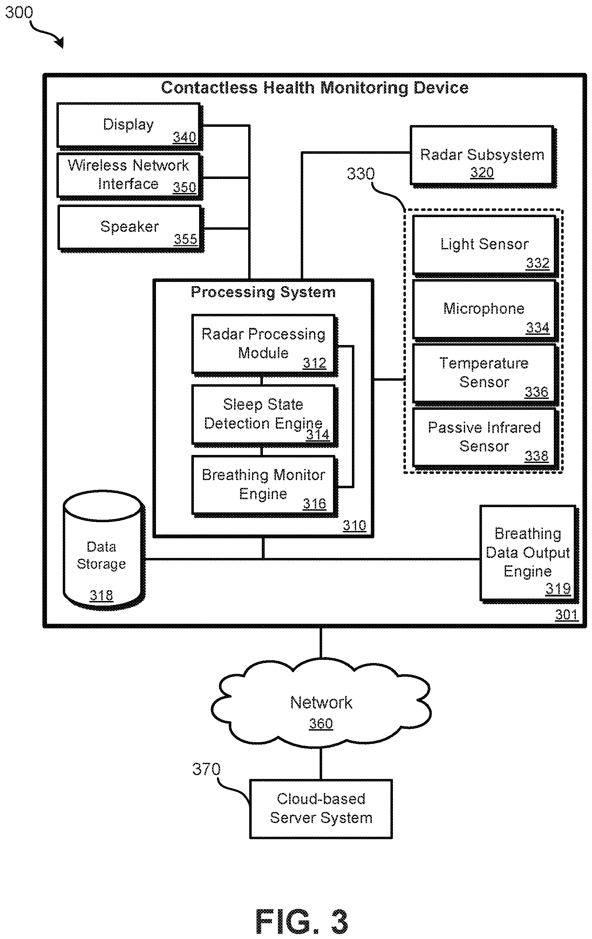

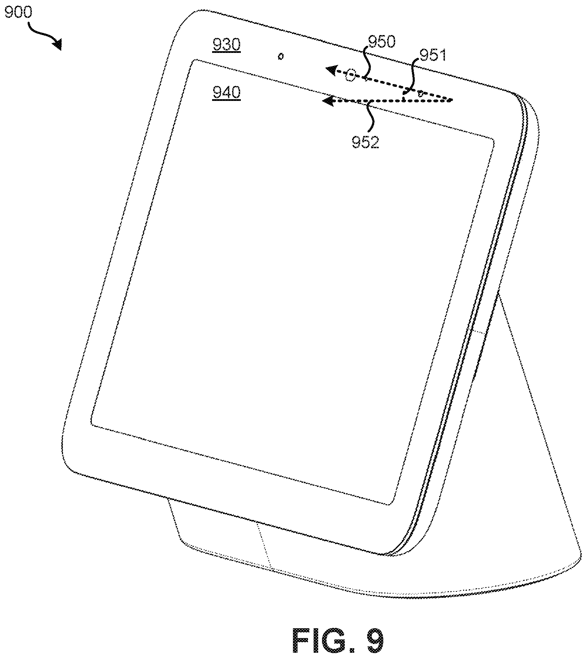

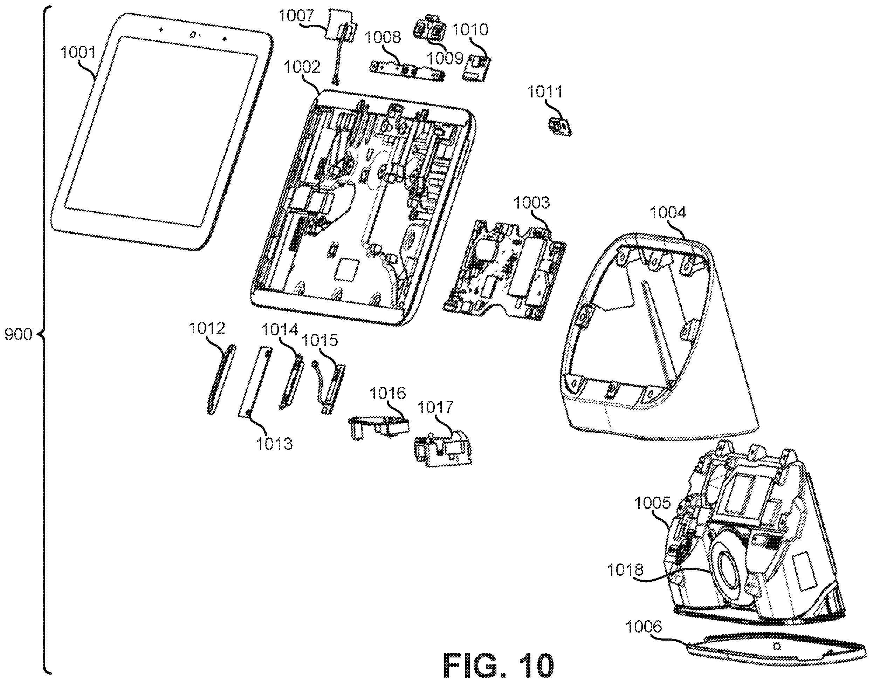

**FIG. 3** illustrates a block diagram of an embodiment of a contactless health monitoring system 300 ("system 300"). System 300 can include: contactless health monitoring device 301 ("device 301"); network 360; and cloud-based server system 370. Device 301 can represent an embodiment of device 210 of FIG. 2. Device 301 can include: processing system 310; data storage 318; radar subsystem 320; environmental sensor suite 330; display 340; wireless network interface 350; and speaker 355. Generally, device 301 can include a housing that houses all of the components of device 301. Further detail regarding such a possible housing is provided in relation to FIG. 9 and FIG. 10.

Processing system 310 can include one or more processors configured to perform various functions, such as the functions of: radar processing module 312; sleep state detection engine 314; and breathing monitor engine 316. Processing system 310 can include one or more special-purpose or general-purpose processors. Such special-purpose processors may include processors that are specifically designed to perform the functions detailed herein. Such special-purpose processors may be ASICs or FPGAs which are general-purpose components that are physically and electrically configured to perform the functions detailed herein. Such general-purpose processors may execute special-purpose software that is stored using one or more non-transitory processor-readable mediums, such as random access memory (RAM), flash memory, a hard disk drive (HDD), or a solid state drive (SSD).

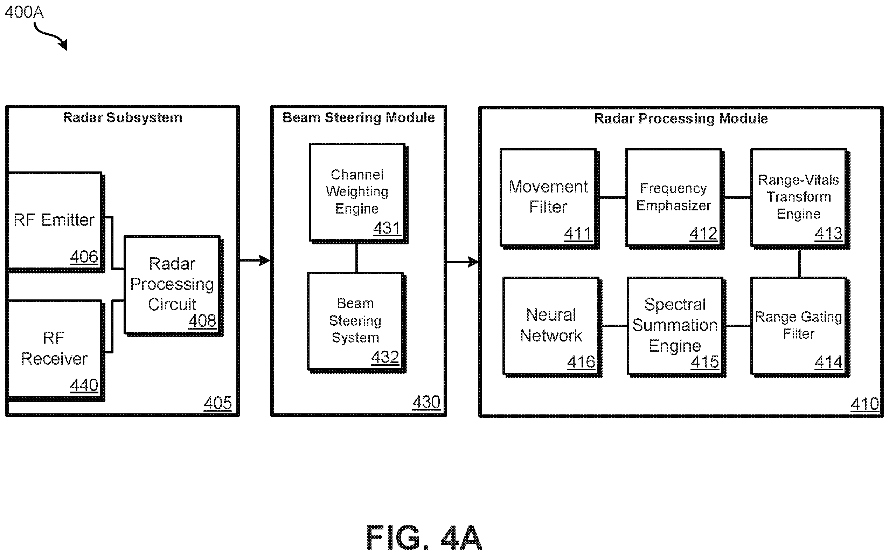

Radar subsystem 320 (also referred to as a radar sensor) can be a single integrated circuit (IC) that emits, receives, and outputs data indicative of a received, reflected waveform. The output of radar subsystem 320 may be analyzed using radar processing module 312 of processing system 310. Further detail regarding radar subsystem 320 and radar processing module 312 is provided in relation to FIG. 4A.

Device 301 may include one or more environmental sensors, such as all, one, or some combination of the environmental sensors provided as part of environmental sensor suite 330. Environmental sensor suite 330 can include:

light sensor 332; microphone 334; temperature sensor 336; and passive infrared (PIR) sensor 338. In some embodiments, multiple instances of some or all of these sensors may be present. For instance, in some embodiments, multiple microphones may be present. Light sensor 332 may be used for measuring an ambient amount of light present in the general environment of device 301. Microphone 334 may be used for measuring an ambient noise level present in the general environment of device 301. Temperature sensor 336 may be used for measuring an ambient temperature of the general environment of device 301. PIR sensor 338 may be used to detect moving living objects (e.g., persons, pets) within the general environment of device 301. Other types of environmental sensors are possible. For instance, a camera and/or humidity sensor may be incorporated as part of environmental sensor suite 330. As another example, active infrared sensors may be included. In some embodiments, some data, such as humidity data, may be obtained from a nearby weather station that has data available via the Internet. In some embodiments, active acoustic sensing methods, included, but not limited to sonar and ultrasound, and including either single or arrayed acoustic sources and/or receivers may be implemented. Such arrangements may be used as one or more adjunct sensing modalities incorporated with the other sensors and methods described herein.

In some embodiments, one, some, or all of sensors of environmental sensor suite 330 may be external to device 301. For instance, one or more remote environmental sensors may communicate with device 301, either directly (e.g., via a direct wireless communication method, via a low-power mesh network) or indirectly (e.g., through one or more other devices via the low-power mesh network, via an access point of a network, via a remote server).

Device 301 may include various interfaces. Display 340, which may be a touchscreen, can allow processing system 310 to present information for viewing by one or more users. Wireless network interface 350 can allow for communication using a wireless local area network (WLAN), such as a WiFi-based network. Speaker 355 can allow for sound, such as synthesized speech, to be output. For instance, responses to spoken commands received via microphone 334 may be output via speaker 355 and/or display 340. The spoken commands may be analyzed locally by device 301 or may be transmitted via wireless network interface 350 to cloud-based server system 370 for analysis. A response, based on the analysis of the spoken command, can be sent back to device 301 via wireless network interface 350 for output via speaker 355 and/or display 340. Additionally or alternatively, the speaker 355 and microphone 334 may be collectively configured for active acoustic sensing, including ultrasonic acoustic sensing. Additionally or alternatively, other forms of wireless communication may be possible, such as using a low-power wireless mesh network radio and protocol (e.g., Thread) to communicate with various smart home devices. In some embodiments, a wired network interface, such as an Ethernet connection, may be used for communication with a network. Further, the evolution of wireless communication to fifth generation (5G) and sixth generation (6G) standards and technologies provides greater throughput with lower latency which enhances mobile broadband services. 5G and 6G technologies also provide new classes of services, over control and data channels, for vehicular networking (V2X), fixed wireless broadband, and the Internet of Things (IoT). Such standards and technologies may be used for communication by device 301.

The low-power wireless mesh network radio and protocol may be used for communicating with power limited devices. A power-limited device may be an exclusively battery powered device. Such devices may rely exclusively on one or more batteries for power and therefore, the amount of power used for communications may be kept low in order to decrease the frequency at which the one or more batteries need to be replaced. In some embodiments, a power-limited device may have the ability to communicate via a relatively high power network (e.g., WiFi) and the low-power mesh network. The power-limited device may infrequently use the relatively high power network to conserve power. Examples of such power-limited devices include environmental sensors (e.g., temperature sensors, carbon monoxide sensors, smoke sensors, motion sensors, presence detectors) and other forms of remote sensors.

Notably, some embodiments of device 301 do not have any still camera or video camera. By not incorporating an on-board camera, users nearby may be reassured about their privacy. For example, device 301 can typically be installed in a user's bedroom. For many reasons, a user would not want a camera located in such a private space or aimed toward the user while the user is sleeping. In other embodiments, device 301 may have a camera, but the camera's lens may be obscured by a mechanical lens shutter. In order to use the camera, the user may be required to physically open the shutter to allow the camera to have a view of the environment of device 301. The user can be assured of privacy from the camera when the shutter is closed.

Wireless network interface 350 can allow for wireless communication with network 360. Network 360 can include one or more public and/or private networks. Network 360 can include a local wired or wireless network that is private, such as a home wireless LAN. Network 360 may also include a public network, such as the Internet. Network 360 can allow for device 301 to communicate with remotely located cloud-based server system 370.

Cloud-based server system 370 can provide device 301 with various services. Regarding breathing data, cloud-based server system 370 can include processing and storage services for breathing-related data. While the embodiment of FIG. 3 involves processing system 310 performing sleep state detection and monitoring a user's breathing, in other embodiments, such functions may be performed by cloud-based server system 370. Also, in addition or in alternate to data storage 318 being used to store breathing data, breathing-related data may be stored by cloud-based server system 370, such as mapped to a common user account to which device 301 is linked. If multiple users are monitored, the breathing data may be stored and mapped to a master user account or to the corresponding users' accounts.

Cloud-based server system 370 may additionally or alternatively provide other cloud-based services. For instance, device 301 may additionally function as a home assistant device. A home assistant device may respond to vocal queries from a user. In response to detecting a vocal trigger phrase being spoken, device 301 may record audio. A stream of the audio may be transmitted to cloud-based server system 370 for analysis. Cloud-based server system 370 may perform a speech recognition process, use a natural language processing engine to understand the query from the user, and provide a response to be output by device 301 as synthesized speech, an output to be presented on display 340, and/or a command to be executed by device 301 (e.g., raise the volume of device 301) or sent to some other smart home device. Further, queries or commands may be submitted to cloud-based server system 370 via display 340, which may be a touchscreen. For instance, device 301 may be used to control various smart home devices or home automation devices. Such commands may be sent directly by device 301 to the device to be controlled or may be sent via cloud-based server system 370.

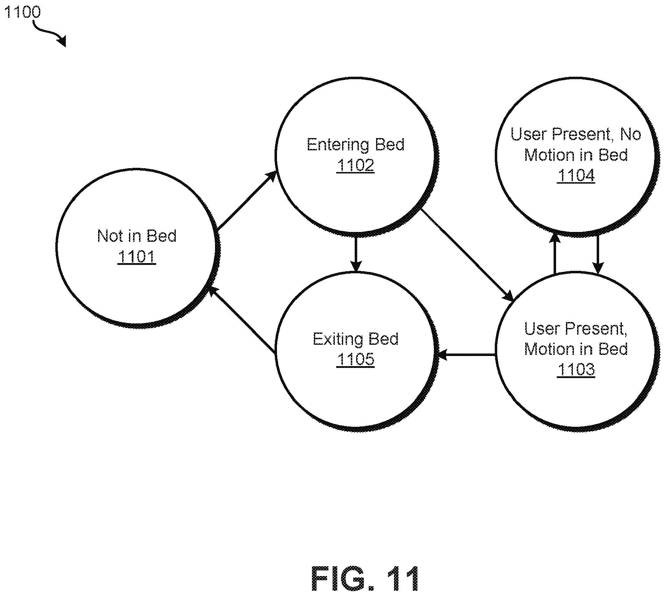

Based on data output by radar processing module 312, sleep state detection engine 314 may be used to determine whether a user is likely asleep or awake. Sleep state detection engine 314 may progress through a state machine, such as detailed in relation to FIG. 11, or may use the state identified using such a state machine to determine whether the user is likely awake or asleep. For example, if a user is determined to be in bed and still for at least a period of time, the user may be identified as asleep. The output of sleep state detection engine 314 may be used by breathing monitor engine 316 to determine when the user's breathing should be analyzed and recorded and/or when breathing monitor engine 316 should be activated or deactivated. For instance, the user's breathing may only be analyzed when the user is asleep and motionless (except for movement due to vital signs).

Data storage 318, which can include one or more non-transitory processor-readable mediums, may store data obtained from breathing monitor engine 316. The data obtained may include indications of a type, when, duration, and/or the intensity of a user-experienced abnormal breathing condition, such as paradoxical breathing. Other breathing-related data may be tracked and stored, such as the user's breathing rate (e.g., breaths per minute). Changes in breathing rate may be monitored nightly and over longer periods of time, such as days, weeks, months, and years. In some embodiments, additionally or alternatively, such data may be stored using cloud-based server system 370.

Breathing data output engine 319 may compile or summarize data received from machine learning model 540 and/or stored by data storage 318 and may output a summary of such data using display 340 and/or speaker 355. For instance, a summary of a user's breathing, including any observed medical issues, may be output in the morning automatically or in response to the user requesting a summary from the previous night. The summary can include a visualization of the user's breathing rate and/or an indication of any identified medical condition (and, possibly, a definition of the identified medical condition). The summary can also include a trend identified over a longer period of time, such as whether the medical condition is abating or intensifying. Some or all of the summary may be output via speaker 355 using synthesized speech. The summary may also be stored locally using data storage 318 or remotely using cloud-based server system 370.

**FIG. 4A** illustrates an embodiment of a contactless health monitoring system 400 ("system 400A") which can perform beam steering. System 400A allows for a contactless health assessment of a user to be made. The contactless health assessment can include a state determination, such as whether the user is present in bed, is motionless within bed, or is moving within bed. The state of the user can be used to determine whether health data about the user should be recorded and/or if other systems should be activated. For instance, system 500B may only be activated if the user is determined to be in bed and motionless. When a user is determined to be in bed and motionless, small movements of the user may be monitored. These small movements of the user's body can correspond to vital signs, such as breathing and heartrate. Such vital sign data may be recorded, stored, and analyzed.

System 400A can include radar subsystem 405 (which can represent an embodiment of radar subsystem 320); radar processing module 410 (which can represent an embodiment of radar processing module 312); and beam steering module 430. Beam steering performed by using beam steering module 430 can focus on radar reflections from a region in which a user may be present and ignore or at least decrease the use of radar reflections from objects that cause interference, such as a nearby wall or other stationary object.

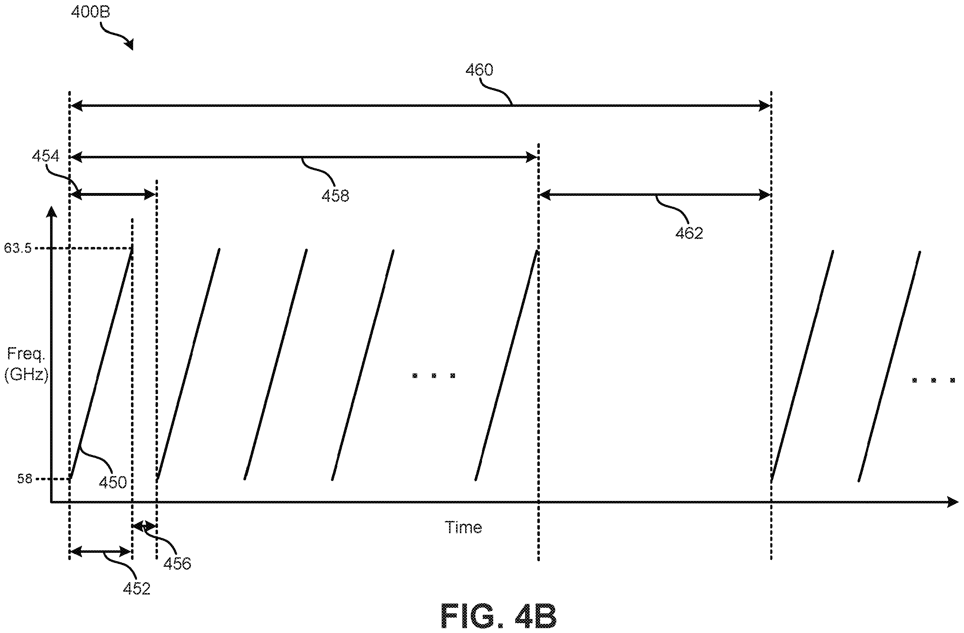

Radar subsystem 405 may include RF emitter 406, RF receiver 407, and radar processing circuit 408. RF emitter 406 can emit radio waves, such as in the form of continuous-wave (CW) radar. RF emitter 406 may use frequency-modulated continuous-wave (FMCW) radar. The FMCW radar may operate in a burst mode, such as detailed in relation to FIG. 4B, with a conversion process being performed to create virtual continuous samples. Radar subsystem 405 may operate in a burst mode or continuous sparse-sampling mode. In burst mode, a frame or burst of multiple chirps, with the chirps spaced by a relatively short period of time, may be output by RF emitter 406. Each frame may be followed by a relatively long amount of time until a subsequent frame. In a continuous sparse-sampling mode, frames or bursts of chirps are not output, rather chirps are output periodically. The spacing of chirps in the continuous sparse sampling mode may greater in duration than the spacing between chirps within a frame of the burst mode. In some embodiments, radar subsystem 405 may operate in a burst mode, but output raw chirp waterfall data for each burst may be combined (e.g., averaged) together to create simulated continuous sparse-sampled chirp waterfall data. In some embodiments, raw waterfall data gathered in burst mode may be preferable for gesture detection while raw waterfall data gathered in a continuously-sparse sampling mode may be preferable for sleep tracking, vital sign detection, and, generally, health monitoring. Gesture detection may be performed by other hardware or software components that use the output of radar subsystem 405 that are not illustrated.

The frequency of radio waves transmitted may repeatedly sweep from a low to high frequency (or the reverse). The power level used for transmission may be very low such that radar subsystem 405 only has an effective range of several meters or an even shorter distance. RF receiver 407 may include one or more antennas and may receive radio wave reflections off of nearby objects of radio waves emitted by RF emitter 406. The reflected radio waves may be interpreted by radar processing circuit 408. Radar processing circuit 408 may output raw waveform data, which can also be referred to as the raw chirp waterfall data for analysis by a separate processing entity. Radar subsystem 405 may be implemented as a single integrated circuit (IC) or radar processing circuit 408 may be a separate component from RF emitter 406 and RF receiver 407. In some embodiments, radar subsystem 405 is integrated as part of device 301 such that RF emitter 406 and RF receiver 407 are pointing in a same direction as display 340. In other embodiments, an external device that includes radar subsystem 405 may be connected with device 301 via wired or wireless communication. For example, radar subsystem 405 may be an add-on device to a home assistant device.

For radar subsystem 405, if FMCW is used, an unambiguous FMCW range can be defined. Within this range, a distance to objects can be accurately determined. However, outside of this range, a detected object could be incorrectly interpreted as nearer than an object within the unambiguous range. This incorrect interpretation can be due to the frequency of the mixed signal and the sampling rate of the ADC used by the radar subsystem to convert the received analog signals to digital signals. If the frequency of the mixed signal is above the Nyquist rate of the sampling of the ADC, the digital data output by the ADC representative of the reflected radar signal can be incorrectly represented (e.g., as a lower frequency indicative of a closer object).

When using device 301 to monitor sleep patterns and vital statistics, a user may be instructed that the user should be the closest person to device 301. It may be possible that another person or an animal is present within the bed. It may be necessary to define the unambiguous FMCW range to be far enough, such as two meters, such that both persons (or, approximately the width of the bed) fall within the unambiguous FMCW range of radar subsystem 405. Two meters may be an ideal distance since this distance is approximately the width of a large commercially available bed (e.g., a king size bed).

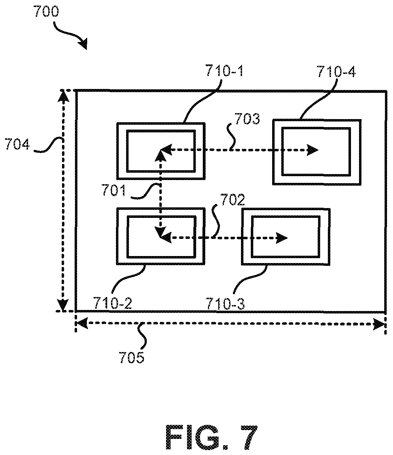

Radar subsystem 405 may contain multiple antennas to receive reflected radar radio waves. In some embodiments, three antennas may be present. These antennas may be aligned in an "L" pattern, such that two antennas are horizontally orthogonal and two antennas are vertically orthogonal with one of the antennas being used in both the horizontal arrangement and vertical arrangement, such as detailed in relation to FIG. 7. By analyzing the phase difference in reflected radio waves, a weighting may be applied to target the received radar beam vertically and/or horizontally. In other embodiments, the antennas may be aligned in a different pattern.

Horizontal beam steering may be performed for multiple reasons. First, horizontal targeting may be performed to compensate for emitted radar being pointed towards an object that causes interference. For instance, if a user's bed headboard is against a wall, the headboard and/or wall may occupy a significant portion of the field-of-view of radar subsystem 405. Radar reflections from the headboard and/or wall might not be used in determining data about the user; therefore, it may be beneficial to deemphasize reflections from the wall and/or headboard and emphasize reflections obtained away from the wall and/or headboard. Therefore, the receive beam may be steered horizontally away from the wall and the headboard by weighting applied to the received radar signals. Second, horizontal beam steering may be performed in order to target multiple spatial zones, such as spatial zones 220 of FIG. 2, thus allowing different portions of a user's chest to be analyzed.

In system 400A, beam steering module 430 is present to perform processing on raw radar waterfall data received from radar subsystem 405 before processing is performed by radar processing module 410. Therefore, beam steering module 430 can function as a preprocessing module prior to the analysis of radar processing module 410 and can serve to emphasize regions where one or more users are expected to be present. Beam steering module 430 may be implemented using hardware, software, or firmware; therefore, beam steering module 430 may be implemented using the same one or more processors as radar processing module 410.

Beam steering module 430 can include channel weighting engine 431 and beam steering system 432. Channel weighting engine 431 can be used to perform a training process to determine a series of weightings to be applied to received radar signals from each antenna prior to the received radar signals being mixed together. Channel weighting engine 431 may perform a training process when a monitored region is determined to be empty. During such time, the strength of signals received from large static objects (e.g., walls, headboards) can be analyzed and weightings can be set to steer the beam horizontally (and possibly vertically) away from such objects. Therefore, the amount of reflection in a static environment may be minimized for a particular distance range (e.g., up to one meter) from the device by channel weighting engine 431 steering the receive radar beam. Such training may also be performed when a user is present. That is, the receive beam of radar subsystem 405 can be steered to where motion is detected, or specifically, to where vital signs of a user are detected.

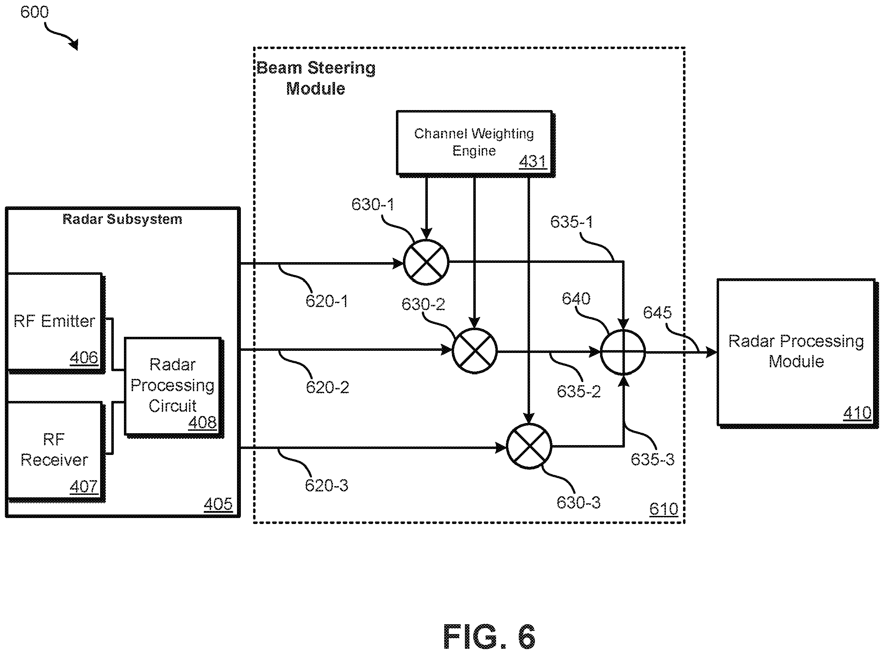

The weightings determined by channel weighting engine 431 may be used by beam steering system 432 to individually apply a weight to the received reflected radar signals of each antenna. The received signals from each antenna may be weighted, then mixed together for processing by radar processing module 410. Further detail regarding how various embodiments of beam steering module 430 may be implemented are detailed in relation to FIGS. 6-8. Beam steering module 430 can be used in combination with any other embodiment detailed herein.

Raw waveform data may be passed from beam steering module 430 to radar processing module 410. Radar processing module 410 may include one or more processors. The functions of radar processing module 410 may be performed using one or more special-purpose or general-purpose processors. Special-purpose processors may include processors that are specifically designed to perform the functions detailed herein. Such special-purpose processors may be ASICs or FPGAs which are general-purpose components that are physically and electrically configured to perform the functions detailed herein. General-purpose processors may execute special-purpose software that is stored using one or more non-transitory processor-readable mediums, such as random access memory (RAM), flash memory, a hard disk drive (HDD), or a solid state drive (SSD). Radar processing module 410 may include: movement filter 411; frequency emphasizer 412; range-vitals transform engine 413; range gating filter 414; spectral summation engine 415; and neural network 416. Each of the components of radar processing module 410 may be implemented using software or firmware, or as specialized hardware.

The raw chirp waterfall or waveform data output by radar subsystem 405 and processed by beam steering module 430 may be received by radar processing module 410 and first processed using movement filter 411. In some embodiments, it is important that movement filter 411 is the initial component used to perform filtering. That is, the processing performed by radar processing module 410 is not commutative in some embodiments. Typically, vital sign determination and sleep monitoring may occur when a monitored user is sleeping or attempting to sleep in a bed. In such an environment, there may typically be little movement. Such movement may be attributed to the user moving within the bed (e.g., rolling over while trying to get to sleep or while asleep) and the user's vital signs, including movement due to breathing and movement due to the monitored user's heartbeat. In such an environment, a large portion of emitted radio waves from RF emitter 406 may be reflected by static objects in the vicinity of the monitored user, such as a mattress, box spring, bed frame, walls, furniture, bedding, etc. Therefore, a large portion of the raw waveform data received from radar subsystem 405 may be unrelated to user movements and the user's vital measurements.

Movement filter 411 may include a waveform buffer that buffers "chirps" or slices of received raw waveform data. For instance, sampling may occur at a rate of 10 Hz. In other embodiments, sampling may be slower or faster. Movement filter 411 may buffer twenty seconds of received raw waveform chirps in certain embodiments. In other embodiments, a shorter or longer duration of buffered raw waveform data is buffered. This buffered raw waveform data can be filtered to remove raw waveform data indicative of stationary objects. That is, for objects that are moving, such as a monitored user's chest, the user's heartbeat and breathing rate will affect the distance and velocity measurements made by radar subsystem 405 and output to movement filter 411. This movement of the user will result in "jitter" in the received raw waveform data over the buffered time period. More specifically, jitter refers to the phase shifts caused by moving objects reflecting emitted radio waves. Rather than using the reflected FMCW radio waves to determine a velocity of the moving objects, the phase shift induced by the motion in the reflected radio waves can be used to measure vital statistics, including heartrate and breathing rate, as detailed herein.

For stationary objects, such as furniture, a zero phase shift (i.e., no jitter) will be present in the raw waveform data over the buffered time period. Movement filter 411 can subtract out such raw waveform data corresponding to stationary objects such that motion-indicative raw waveform data is passed to frequency emphasizer 412 for further analysis. Raw waveform data corresponding to stationary objects may be discarded or otherwise ignored for the remainder of processing by radar processing module 410.

In some embodiments, an infinite impulse response (IIR) filter is incorporated as part of movement filter 411. Specifically, a single-pole IIR filter may be implemented to filter out raw waveform data that is not indicative of movement. Therefore, the single-pole IIR filter may be implemented as a high-pass, low-block filter that prevents raw waveform data indicative of movement below a particular frequency from passing through to frequency emphasizer 412. The cut-off frequency may be set based on known limits to human vital signs. For example, a breathing rate may be expected to be between 10 and 60 breaths per minute. Movement data indicative of a lower frequency than 10 breaths per minute may be excluded by the filter. In some embodiments, a band-pass filter may be implemented to exclude raw waveform data indicative of movement at high frequencies that are impossible or improbable for human vital signs. For instance, a heartrate, which can be expected to be above a breathing rate, may be unlikely to be above 150 beats per minute for a person in a resting or near-resting state. Raw waveform data indicative of a higher frequency may be filtered out by the band pass filter.

In some embodiments, it may be possible to further fine-tune the frequencies of raw waveform data that movement filter 411 passes to frequency emphasizer 412. For instance during an initial configuration phase, a user may provide information about the monitored user (e.g., himself, a child), such as age data. Table 1 indicates typical respiratory rates for various ages. Similar data may be present for heartrate. The filter may be configured to exclude data that is outside of the expected breathing rate, heartrate range, or both.

**Table 1**

| Age | Breathing Rate Range (breaths per minute) |
| --- | --- |
| Birth - 6 weeks | 30-60 |
| 6 months | 25-40 |
| 3 years | 20-30 |
| 6 years | 18-25 |
| 10 years | 17-23 |
| Adults | 12-18 |
| 65-80 years old | 12-28 |
| > 80 years old | 10-30 |

The vital signs of the monitored user being measured are periodic impulse events: a user's heartrate may vary over time, but it can be expected that the user's heart will continue to beat periodically. This beating is not a sinusoidal function, but rather may be understood as an impulse event, more analogous to a square wave having a relatively low duty cycle that induces motion in the user's body. Similarly, a user's breathing rate may vary over time, but breathing is a periodic function performed by the user's body that is analogous to sinusoidal function, except that a user's exhale is typically longer than their inhale. Further, at any given time, a particular window of waveform data is being analyzed. Since a particular time window of waveform data is being analyzed, even a perfect sinusoid within that window can result in spectral leakage in the frequency domain. Frequency components due to this spectral leakage should be deemphasized.

Frequency emphasizer 412 may work in conjunction with range-vitals transform engine 413 to determine the one (e.g., breathing) or two (e.g., breathing plus heartbeat) frequency components of the raw waveform data. Frequency emphasizer 412 may use frequency windowing, such as a 4D Hamming window (other forms of windowing are possible, such as a Hann window), to emphasize important frequency components of the raw waveform data and to deemphasize or remove waveform data that is attributable to spectral leakage outside of the defined frequency window. Such frequency windowing may decrease the magnitude of raw waveform data that is likely due to processing artifacts. The use of frequency windowing can help reduce the effects of data-dependent processing artifacts while preserving data relevant for being able to separately determine heartrate and breathing rate.

For a stationary bedside FMCW radar-based monitoring device, which may be positioned within 1 to 2 meters of the one or more users being monitored to detect breathing and heartrate (e.g., using radar as emitted in **FIG. 4B**), a 2D Hamming window that emphasizes frequencies in the range of 10 to 60 bpm (0.16 Hz to 1 Hz) for breathing and 30 to 150 bpm (0.5 to 2.5 Hz) for heartbeat provide for sufficiently good signals to make reliable measurements without requiring advance knowledge of the subject's age or medical history.

Since heartrate and breathing rate are periodic impulse events, the frequency domain heartrate, and breathing rate may be represented by different fundamental frequencies, but each may have many harmonic components at higher frequencies. One of the primary purposes of frequency emphasizer 412 may be to prevent the frequency ripples of harmonics of the monitored user's breathing rate from affecting the frequency measurement of the monitored user's heartrate (or the reverse). While frequency emphasizer 412 may use a 2D Hamming window, it should be understood that other windowing functions or isolating functions can be used to help isolate frequency ripples of the monitored user's breathing rate from the frequency ripples of the monitored user's heartrate.

Range-vitals transform engine 413 analyzes the received motion-filtered waveform data to identify and quantify the magnitude of movement at specific frequencies. More particularly, range-vitals transform engine 413 analyzes phase jitter over time to detect relatively small movements due to a user's vital signs that have a relatively low frequency, such as breathing rate and heart rate. The analysis of range-vitals transform engine 413 may assume that the frequency components of the motion waveform data are sinusoidal. Further, the transform used by range-vitals transform engine 413 can also identify the distance at which the frequency is observed. Frequency, magnitude, and distance can all be determined at least in part because radar subsystem 405 uses an FMCW radar system.

Prior to applying the transform of range-vitals transform engine 413, a zero-padding process may be performed by range-vitals transform engine 413 to add a number of zeros to the motion-filtered raw waveform data. By performing a zero-padding process, the resolution within the frequency domain can be increased effectively, allowing for more accurate low-rate measurements (e.g., a low heartrate, a low breathing rate). For example, zero-padding can help numerically increase resolution to detect differences of half a breath per minute compared to a resolution of a breath per minute without zero-padding. In some embodiments, three to four times the number of zeros compared to the buffered sample size of the raw waveform data may be added. For example, if twenty seconds of buffered raw waveform data are analyzed, sixty to eighty seconds' worth of zero padding may be added to the sample. Specifically, the range of three to four times zero padding of the sample was found to substantially increase resolution while not making the transform process overly complex (and, thus, processor use-intensive).

In order to determine the amount of zero padding to be performed, equations 1-3 may be used. In equation 1, *RPM_resolution* may ideally be less than 1.

$$RPM_{resolution} = 60 * \frac{chirp\_rate}{n\_fft\_slow\_time} \qquad \textbf{Eq. 1}$$

$$n\_FFT\_slow\_time\_min = (nearest\_power\_of\_2)(60 * chirp\_rate) \qquad \textbf{Eq. 2}$$

In some embodiments, a chirp rate (*chirp_rate*) of 30 Hz may be used. Such a frequency may have sufficient margin from Nyquist limits of the upper limit of breathing rate and heartbeat rate. *n_FFT_slow_time_min* may, therefore, be 2048. Given a 20 second window for estimating respiration statistics, Equation 3 results in a value of 600.

$$n\_chirps\_for\_respiration = 20 * chirp\_rate = 600 \qquad \textbf{Eq. 3}$$

This value of 600 is smaller than the required vitals-FFT size and makes range-vitals transform engine 413 perform a 3x to 4x zero padding. A balance in how much zero padding to perform may be based on increasing the frequency resolution and associated increases in the amount of computation needed to perform the FFT. A 3x to 4x zero padding has been found to provide sufficient resolution for heartrate and breath rate while moderating the amount of computation needing to be performed.

Range-vitals transform engine 413 can perform a series of Fourier transform (FT) to determine the frequency components of the received raw waveform data output by frequency emphasizer 412. Specifically, a series of fast Fourier transform (FFT) may be performed by range-vitals transform engine 413 to determine the specific frequencies and magnitudes of waveform data at such frequencies.

Waveform data obtained over a period of time can be expressed in multiple dimensions. A first dimension (e.g., along the y-axis) can relate to multiple samples of waveform data from a particular chirp and a second dimension (e.g., along the x-axis) relates to a particular sample index of waveform data gathered across multiple chirps. A third dimension of data (e.g., along the z-axis) is present indicative of the intensity of the waveform data.

Multiple FFTs may be performed based on the first and second dimension of the waveform data. FFTs may be performed along each of the first and second dimensions: an FFT may be performed for each chirp and an FFT may be performed for each particular sample index across multiple chirps that occurred during the period of time. An FFT performed on waveform data for a particular reflected chirp can indicate one or more frequencies, which, in FMCW radar, are indicative of the distances at which objects are present that reflected emitted radio waves. An FFT performed for a particular sample index across multiple chirps can measure the frequency of phase jitter across the multiple chirps. Therefore, the FFT of the first dimension can provide the distance at which a vital statistic is present and the FFT of the second dimension can provide a frequency of the vital statistic. The output of the FFTs performed across the two dimensions is indicative of: 1) the frequencies of vital statistics; 2) the ranges at which the vital statistics were measured; and 3) the magnitudes of the measured frequencies. In addition to values due to vital statistics being present in the data, noise may be present that is filtered, such as using spectral summation engine 415. The noise may be partially due to heartrate and breathing not being perfect sinusoidal waves.

To be clear, the transform performed by range-vitals transform engine 413 differs from a range-Doppler transform. Rather than analyzing changes in velocity (as in a range-Doppler transform), periodic changes in phase shift over time are analyzed as part of the range-vitals transform. The range-vitals transform is tuned to identify small movements (e.g., breathing, heart rate) occurring over a relatively long period of time by tracking changes in phase, referred to as phase jitter. As previously detailed, zero padding is performed to allow for sufficient resolution for accurate determination of heartrate and breathing rate.

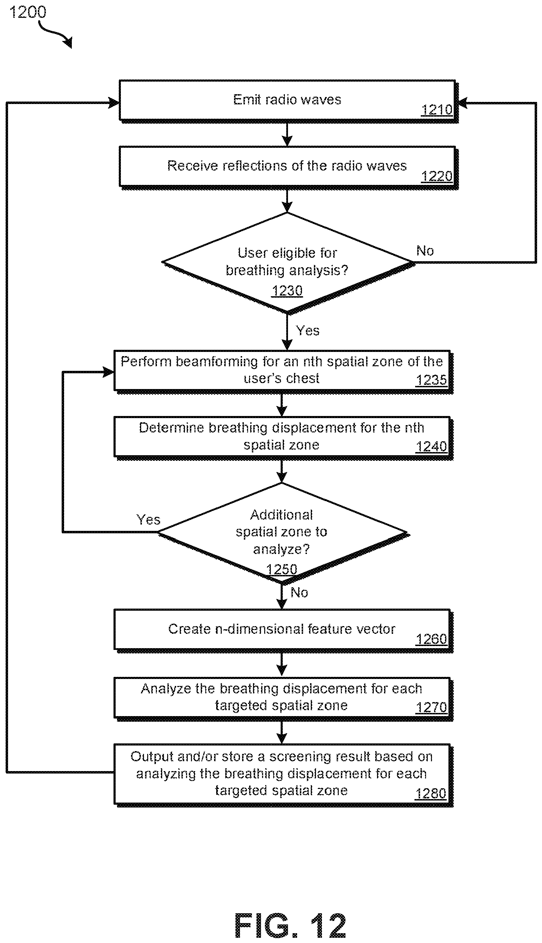

Range gating filter 414 is used to monitor a defined range of interest and exclude waveform data due to movement beyond the defined range of interest. For arrangements detailed herein, the defined range of interest may be 0 to 1 meter. In some embodiments, this defined range of interest may be different or possibly set by a user (e.g., via a training or setup process) or by a service provider. In some embodiments, a goal of this arrangement may be to monitor the one person closest to the device (and exclude or segregate data for any other person farther away, such as a person sleeping next to the person being monitored). In other embodiments, if both persons are to be monitored, the data may be segregated, as detailed in relation to **FIG. 12.** Therefore, range-vitals transform engine 413 and range gating filter 414 serve to segregate, exclude, or remove movement data attributed to objects outside of the defined range of interest and sum the energy of movement data attributed to objects within the defined range of interest. The output of range gating filter 414 may include data that has a determined range within the permissible range of range gating filter 414. The data may further have a frequency dimension and a magnitude. Therefore, the data may possess three dimensions.

Spectral summation engine 415 may receive the output from range gating filter 414. Spectral summation engine 415 may function to transfer the measured energy of harmonic frequencies of the heartrate and breathing rate and sum the harmonic frequency energy onto the fundamental frequency's energy. This function can be referred to as a harmonic sum spectrum (HSS). Heartrate and breathing rate are not sinusoidal; therefore, in the frequency domain, harmonics will be present at frequencies higher than the fundamental frequency of the user's breathing rate and the fundamental frequency of the user's heartrate. One of the primary purposes of spectral summation engine 415 is to prevent harmonics of the monitored user's breathing rate from affecting the frequency measurement of the monitored user's heartrate (or the reverse). The HSS may be performed at the second order by summing the original spectrum with a downsampled instance (by a factor of two) of the spectrum. This process may also be applied at higher order harmonics such that their respective spectra are added to the spectrum at the fundamental frequency.

At this stage, for a person in bed who is lying still (with the exception of movement due to breathing and heartrate), it will be expected that two major frequency peaks will present in the frequency data. However, if the monitored user is physically moving, such as rolling over in bed, the energy will be significantly distributed across the frequency spectrum (a broader distribution). Such large physical movement may manifest itself in the frequency data as being a large number of small peaks. If the bed is empty, rather than a person being present, there may be no or almost no frequency components above the noise floor since movement filter 411 has previously filtered raw waveform data corresponding to static objects. The distribution and magnitude of frequency peaks across the spectrum may be used to determine if the user is likely awake or asleep.

Spectral summation engine 415 may output a feature vector that is indicative of heartrate (e.g., in beats per minute) and breathing rate (e.g., in breaths per minute). The feature vector can indicate frequency and magnitude. Neural network 416 may be used to determine whether the heartrate and/or breathing rate indicated in the output of the feature vector from spectral summation engine 415 should be considered valid. Therefore, the heartrate and breathing rate output by spectral summation engine 415 may be stored, presented to a user, and/or treated as valid based on the output of neural network 416. Neural network 416 may be trained (e.g., using supervised learning performed using a training set of data) to output one of three states, such as those indicated in Table 2 by performing a spectral analysis. Vital statistic data may be considered valid when the user is determined to be present and the detected movement is due to the user's vital signs.

Each state in Table 2 is associated with a different spectral energy and spectral sparsity profile. Spectral energy refers to a summation of the energy across the frequency spectrum detected due to motion being present within the monitored region. Spectral sparsity represents whether movement tends to be distributed across a wide range of frequencies or clustered at a few specific frequencies. For instance, if energy peaks occur at few frequencies, such as when the user's vital signs are detected (but not other movement), spectral sparsity is high. However, if peaks (over a threshold value) or some other form of determination based on a threshold criterion at least partially based on magnitude) occur at many frequencies, spectral sparsity is low.

As an example, motion due to a vital sign, such as a heartbeat, may be indicative of significant movement (e.g., high spectral energy) at specific frequencies (e.g., high spectral sparsity); motion due to a user moving a limb may also be indicative of significant movement (high spectral energy), but may have low spectral sparsity. The neural network may be trained to distinguish between each state based on the spectral energy profile output by spectral summation engine 415. Therefore, neural network 416 may be provided two features, a first value representing spectral energy and a second value representing spectral sparsity.

The output of spectral summation engine 415 may be characterized as a feature vector having a first dimension of frequency and a second dimension of amplitude. The first value representing spectral energy may be calculated by determining the maximum amplitude present in the feature vector output by spectral summation engine 415. This maximum amplitude value may be normalized to a value within 0 to 1. The second value representing the spectral sparsity may be calculated by subtracting the median amplitude of the feature vector from the maximum amplitude. Again here, the calculated sparsity may be normalized to a value within 0 to 1.

Table 2 represents a generalization of how the features of spectral energy and spectral sparsity is used as features by the trained neural network to classify the state of the monitored region.

**Table 2**

| State of Monitored Region | Spectral Energy | Spectral Sparsity |
|---|---|---|
| User present and vitals-only movement | High | High |
| User present and moving (limb and torso movements) | High | Low |
| No user present | Low | Low |

The state of the monitored region classified by neural network 416 may be used in determining the monitored user's sleep state or, more generally, whether the user is moving or still within bed. The state of the monitored region as determined by the performed classification of neural network 416 may further be used to determine if the vital statistics output by spectral summation engine 415 should be trusted or ignored. For accurate vital statistic determination, heartrate and breathing rate may be identified as likely accurate when neural network 416 determines that the user is present and still (i.e., no large physical movements; however, movement is occurring due to breathing and/or heartbeat). In some embodiments, the vital statistics output by spectral summation engine 415 may be exclusively stored locally (e.g., to alleviate privacy concerns); in other embodiments, the vital statistics output may be transmitted to cloud-based server system 370 for remote storage (in alternative or in addition to such data being stored locally).

Neural network 416 may be initially trained using a large set of training data of amplitude and frequency feature vectors that have been properly tagged with a classification as mapping the spectral energy and the spectral sparsity to the corresponding ground-truth state of the monitored region. Alternatively, neural network 416 may be initially trained using a large set of training data of amplitude and frequency feature vectors that has been properly classified as mapping the comprising spectral energy and the spectral sparsity pairs each properly tagged to the corresponding ground-truth state of the monitored region. The neural network may be a fully connected neural network that is not time-dependent. In some embodiments, a machine-learning arrangement, classifier, or form of artificial intelligence other than a neural network may be used.

In other embodiments, rather than a spectral energy value and a spectral sparsity value being the features used by the neural network, a neural network, possibly with extra front-end convolutional layers, can be trained to use the output of range gating filter 414 directly. Rather, an embodiment of a convolutional network can analyze the frequency and magnitude data output by range gating filter 414 to classify the state of the user. The convolutional neural network may be trained to utilize offline training that is based on a set of spectral measurements mapped to the ground truth state of the monitored region prior to system 400A being used by an end user.

The sleep state determined by neural network 416 may be stored, along with time data, to data storage 318. The vital statistics output by spectral summation engine 415 can be stored to a vital statistic datastore when neural network 416 indicates that the monitored user is present and still. Other vital statistic data may be discarded or possibly flagged to indicate it is less likely to be correct. The data stored to data storage 318 and a vital statistic datastore may be stored locally at device 301. In some embodiments, storage occurs at only device 301. Such an implementation may help alleviate a concern about health-related data being transmitted and stored remotely. In some embodiments, the monitored user may elect to have sleep data and vital statistic data transmitted via a network interface (e.g., wireless network interface 350), stored, and analyzed externally, such as by cloud-based server system 370. Storage by cloud-based server system 370 may have significant benefits, such as the ability for the user to access such data remotely, allow access to a medical provider, or participate in research studies. The user may retain the ability to delete or otherwise remove the data from cloud-based server system 370 at any time.

In some embodiments, radar processing module 410 may be wholly or partly located remotely from device 301. While radar subsystem 405 may need to be local to the monitored user, the processing of radar processing module 410 may be moved to cloud-based server system 370. In other embodiments, a smart home device that is in local communication (e.g., via a LAN or WLAN) with device 301 may perform some or all of the processing of radar processing module 410. In some embodiments, a local communication protocol, such as involving a mesh network, can be used to transmit the raw waveform data to the local device that will be performing the processing. Such communication protocols can include Wi-Fi, Bluetooth, Thread, or communication protocols of the IEEE 802.11 and 802.15.4 families. Similar to the processing, storage of the sleep data and vital statistic data may occur at cloud-based server system 370 or another smart home device in the home at which device 301 is located. In still other embodiments, radar processing module 410 may be incorporated with radar subsystem 405 as a single component or system of components.

The stored sleep data of data storage 318 and the vital statistic data may be used to provide the user with short-term and long-term trends relating to their sleeping patterns, vital statistics, or both by sleep data compilation engine 319. For instance, each morning, graphs, statistics, and trends may be determined by sleep data compilation engine 319 based on data stored to data storage 318 and output for display by sleep data compilation engine 319 via display 340. A graph that is indicative of sleep data from the previous night and possibly one or more graphs indicative of breathing rates and heartrate during the previous night may be presented. Similar graphs, trends, and statistics may be output for significantly longer periods of time, such as weeks, months, years, and even multi-year stretches of time by sleep data compilation engine 319. Other uses for sleep data and vital statistics may be possible. For instance, if certain triggers regarding heartrate, breathing rate, and/or sleeping patterns are triggered, a medical professional may be notified. Additionally or alternatively, a notification may be output to the user indicating that the collected data is potentially concerning or is indicative of a healthy person. In some instances, specific sleep problems may be identified, such as sleep apnea. Sleep data may be output via speaker 355 using synthesized speech (e.g., in response to the user waking, in response to a spoken user command, or in response to a user providing input via a touchscreen, such as display 340). Such sleep data may also be represented graphically and or textually on display 340.

System 400A may additionally include beam steering module 430. Beam steering module 430 may include channel weighting engine 431, which may be implemented similarly to the components of radar processing module 410 using software, firmware, and/or hardware. Beam steering module 430 is illustrated as separate from radar processing module 410 because it processes data received from radar subsystem 405 to emphasize data received from a particular direction and deemphasize data received from other directions. Beam steering module 430 may be implemented using the same hardware as radar processing module 410. For instance, beam steering module 430 may be a software process that modifies the radar data received from radar subsystem 405 prior to movement filter 411 being applied. Device 301 may be a surface-top device that is intended to be placed in a particular location, connected with a continuous power supply (e.g., a household power outlet) and interacted with via voice and/or a touchscreen. Therefore, radar subsystem 405 may remain pointed at a portion of the ambient environment for significant periods of time (e.g., multiple hours, days, weeks, months). Generally speaking, beam steering module 430 may be used to map the environment (e.g., room) in which device 301 is located and steer the sensing direction of radar subsystem 405 to a zone within the field-of-view of radar subsystem 405 most likely to have a user present.

Targeting the region within the field-of-view of radar subsystem 405 may help decrease an amount of false negatives and false positives caused by movement of objects other than a user. Further, targeting can help compensate for an angle and location of device 301 relative to where the user sleeps. (For instance, device 301 may be located on a nightstand that is at a different height than the user's bed. Additionally or alternatively, radar subsystem 405 device 301 might not be pointed directly at the location in the bed where the user sleeps.)

When no user is determined to be present, such as based on low spectral energy and low spectral density of Table 2, an optimal beam steering process may be performed by channel weighting engine 431 and beam steering system 432. While no user is present, an analysis can be performed to determine which directional alignment of radar subsystem 405 provides minimalclutter.

FIG. 4B illustrates an embodiment of chirp timing diagram 400B for frequency modulated continuous wave (FMCW) radar radio waves output by a radar subsystem. Chirp timing diagram 400B is not to scale. Radar subsystem 405 may generally output radar in the pattern of chirp timing diagram 400B. Chirp 450 represents a continuous pulse of radio waves that sweeps up in frequency from a low frequency to a high frequency. In other embodiments, individual chirps may continuously sweep down from a high frequency to a low frequency, from a low frequency to a high frequency, and back to a low frequency, or from a high frequency to a low frequency and back to a high frequency. In some embodiments, the low frequency is 58 GHz and the high frequency is 63.5 GHz. (For such frequencies, the radio waves may be referred to as millimeter waves.) In some embodiments, the frequencies are between 57 and 64 GHz. The low frequency and the high frequency may be varied by embodiment. For instance, the low frequency and the high frequency may be between 45 GHz and 80 GHz. The frequencies select may be selected at least in part to comply with governmental regulation. In some embodiments, each chirp includes a linear sweep from a low frequency to a high frequency (or the reverse). In other embodiments, an exponential or some other pattern may be used to sweep the frequency from low to high or high to low.

Chirp 450, which can be representative of all chirps in chirp timing diagram 400B, may have chirp duration 452 of 128 $\mu$s . In other embodiments, chirp duration 452 may be longer or shorter, such as between 30 $\mu$s and 600 $\mu$s. In some embodiments, a period of time may elapse before a subsequent chirp is emitted. Inter-chirp pause 456 may be 205.33 $\mu$s. In other embodiments, inter-chirp pause 456 may be longer or shorter, such as between 10 $\mu$s and 1 ms. In the illustrated embodiment, chirp period 454, which includes chirp 450 and inter-chirp pause 456, may be 333.33 $\mu$s or 500 $\mu$s. This duration varies based on the selected chirp duration 452 and inter-chirp pause 456.

A number of chirps that are output, separated by inter-chirp pauses may be referred to as burst 458 or frame 458. Frame 458 may include twenty chirps. In other embodiments, the number of chirps in frame 458 may be greater or fewer, such as between 1 and 100. The number of chirps present within frame 458 may be determined based an average power that is to be radiated. The FCC or other regulatory agency may set a maximum amount of power that is permissible to be radiated into an environment on average. For example, a duty cycle requirement may be present that limits the duty cycle to less than 10% for any 33 ms time period. In one particular example in which there are twenty chirps per frame, each chirp can have a duration of 128 us, and each frame being 33.33 ms in duration. The corresponding duty cycle is (20 frames)*(.128 ms)/(33.33 ms), which is about 7.8%. By limiting the number of chirps within frame 458 prior to an inter-frame pause, the total amount of power output may be limited. In some embodiments, the peak EIRP (effective isotropically radiated power) may be 13 dBm (20 mW) or less, such as 12.86 dBm (19.05 mW). In other embodiments, the peak EIRP is 15 dBm or less and the duty cycle is 15% or less. In some embodiments, the peak EIRP is 20 dBm or less. That is, at any given time, the average amount of power radiated by the radar subsystem might never exceed such values. Further, the total power radiated over a period of time may be limited. In some embodiments, a duty cycle might not be required.

Frames may be transmitted at a frequency of 30 Hz (33.33 ms) as shown by time period 460. In other embodiments, the frequency may be higher or lower. The frame frequency may be dependent on the number of chirps within a frame and the duration of inter-frame pause 462. For instance, the frequency may be between 1 Hz and 50 Hz. In some embodiments, chirps may be transmitted continuously, such that the radar subsystem outputs a continuous stream of chirps interspersed with inter-chirp pauses. Tradeoffs can be made to save on the average power consumed by the device due to transmitting chirps and processing received reflections of chirps. Inter-frame pause 462 represents a period of time when no chirps are output. In some embodiments, inter-frame pause 462 is significantly longer than the duration of frame 458. For example, frame 458 may be 6.66 ms in duration (with chirp period 454 being 333.33 $\mu$s and 20 chirps per frame). If 33.33 ms occur between frames, inter-frame pause 462 may be 26.66 ms. In other embodiments, the duration of inter-frame pause 462 may be larger or smaller, such as between 15 ms and 40 ms.

In the illustrated embodiment of **FIG. 4B,** a single frame 458 and the start of a subsequent frame are illustrated. It should be understood that each subsequent frame can be structured similarly to frame 458. Further, the transmission mode of the radar subsystem may be fixed. That is, regardless of whether a user is present or not, the time of day, or other factors, chirps may be transmitted according to chirp timing diagram 400B. Therefore, in some embodiments, the radar subsystem always operates in a single transmission mode, regardless of the state of the environment or the activity attempting to be monitored. A continuous train of frames similar to frame 458 may be transmitted while device 301 is powered on.

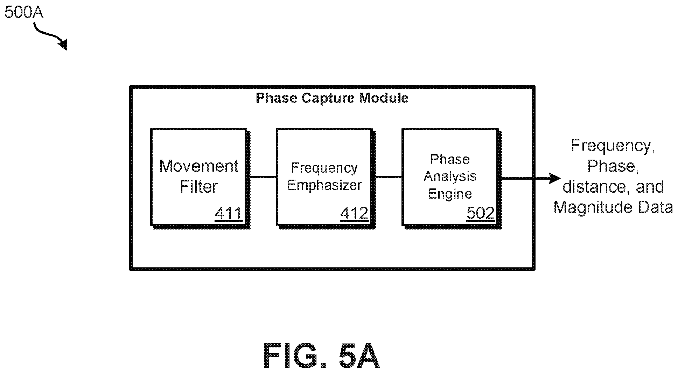

**FIG. 5A** illustrates an embodiment of a phase capture module 500A that may be used as part of a contactless health monitoring system. Phase capture module 500A may be used as part of contactless health monitoring system 500B. Phase capture module 500A can represent a modified embodiment of radar processing module 410. Phase capture module 500A can include movement filter 411, frequency emphasizer 412, and phase analysis engine 502. Each of these components may be implemented using software processes executed by a general-purpose or specialized processor (or multiple processors). Specialized hardware may be implemented to perform the functionality of phase capture module 501. Movement filter 411 and frequency emphasizer 412 can function as detailed in relation to radar processing module 410 of FIG. 4A. For phase capture module 500A, frequency emphasizer 412 can be configured to emphasize frequency components attributed to breathing (and, possibly, deemphasize frequencies related to heartrate).

Phase analysis engine 502 may generally serve to output an instantaneous indication of phase present in the waveform data output from a beam steering module for a particular reflected radar chirp. On the waveform data received by phase analysis engine 502, a fast Fourier transform (FFT) can be performed to determine a phase value (in reference to an absolute phase value) and magnitude for each frequency component of a reflected chirp. For each reflected chirp in the waveform data, phase analysis engine 502 outputs data indicative of frequencies, distances at which the frequencies were detected, phases of the frequency components, and magnitudes of the frequency components.

Radar subsystem 405 can include an analog to digital converter (ADC) to convert the received reflected waveform data to digital waveform data. (FMCW can also include mixing being performed prior to the analog to digital conversion in which a received reflected waveform is mixed with a waveform being transmitted.) Since all ADCs have a finite resolution, a change in distance to a target moving object (e.g., a user's chest) sufficient enough to cause a change in phase such that the difference in phase is observable in the digital data output by the ADC may be needed. Equation 2 indicates the effect of distance that a moving object has on phase.

$$\Phi_{\text{tone}} = 2\pi \left( f_0 * \frac{2R}{c} - \frac{1}{2} * \frac{f_1 - f_0}{T_{chirp}} * \left( \frac{2R}{c} \right)^2 \right) \qquad \textbf{Eq. 4}$$

In Equation 4, for FMCW, $f_0$ represents the lowest frequency of a chirp; $f_1$ represents the highest frequency of a chirp, $c$ represents the speed of light, and $R$ represents the range (distance) to the moving object from the radar sensor. $T_{chirp}$ represents the time duration, in seconds, of a single emitted chirp. A relatively small change in $R$ over the frequency range used for chirps as detailed in relation to FIG. 4B (such as due to movement of a user's chest during breathing), results in a change in phase that is substantial enough that it can be detected in the digital data output by the ADC as part of the digital waveform data. Therefore, Equation 4 confirms that phase can be used to accurate measure chest displacement of a user due to breathing.

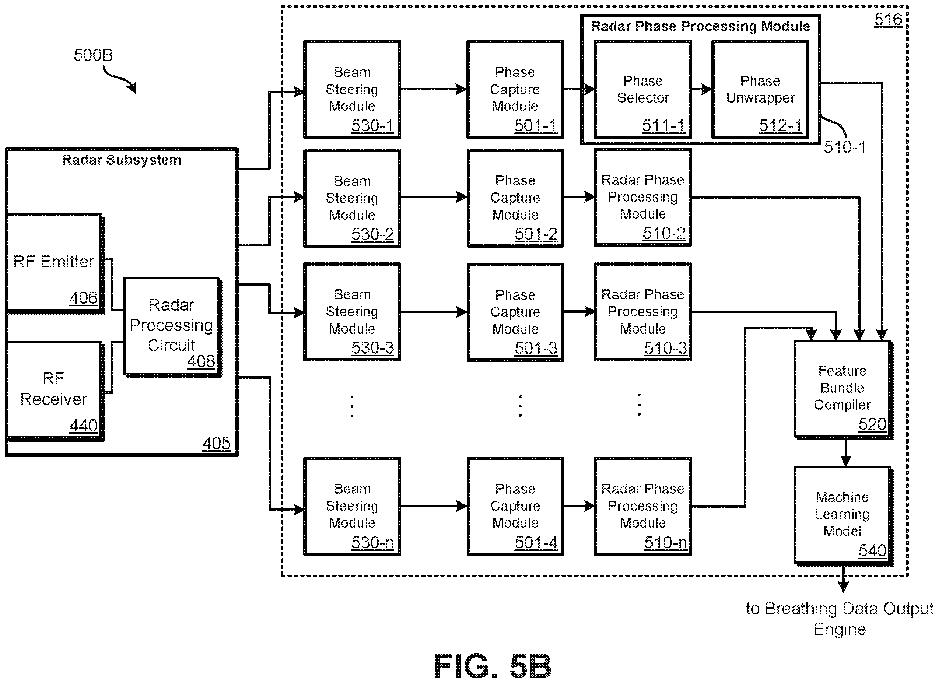

**FIG. 5B** illustrates an embodiment of a contactless health monitoring system 500B ("system 500B") that monitors multiple spatial zones. System 500B can utilize the same output of radar subsystem 405 as system 400A. Components 516 may correspond to breathing monitor engine 316 of FIG. 3. In some embodiments, a user is determined to be in bed and motionless for at least an amount of time or some other time-based criterion prior to components 516 being activated and executed using one or more processors. Components 516 may be performed using one or more general purpose processors executing specialized software. In other embodiments, one or more specialized processors may be configured to perform the functions of system 500B.

Each spatial zone monitored may correspond to a different targeted portion of a user's chest, such as spatial zones 220 in FIG. 2. System 500B can include: radar subsystem 405; beam steering module 430; phase capture modules 501; radar phase processing modules 510; feature bundle compiler 520; and machine learning model 540.

Radar subsystem 405 functions as detailed in relation to FIG. 4A. Radar subsystem 405 includes multiple antennas that are used to receive reflected radar chirps. Separate waveform data is output for each receive antenna of radar subsystem 405. In FIG. 4A, a single beam steering module 430 is present. By a single beam steering module being present, a single direction may be targeted. Beam steering module 430 may determine a general spatial zone that includes the user's chest. Once the general spatial zone has been determined, this region may be more finely parsed by beam steering toward multiple spatial zones within the general spatial zone. In contrast, multiple receive-side digital beam steering modules 530 are present in system 500. Each of these beam steering modules may function as detailed in relation to beam steering module 430. Each of beam steering modules 530 may use different weightings to beam-steer toward different portions of a user's chest. For instance, referring to FIG. 2, beam steering module 530-1 may use weightings to focus on spatial zone 220-1, beam steering module 530-2 may use different weightings to focus on spatial zone 220-2, and so on. Depending on embodiment, various numbers of beam steering modules 530 are present, such as two, three, four, five, or more targeting modules.

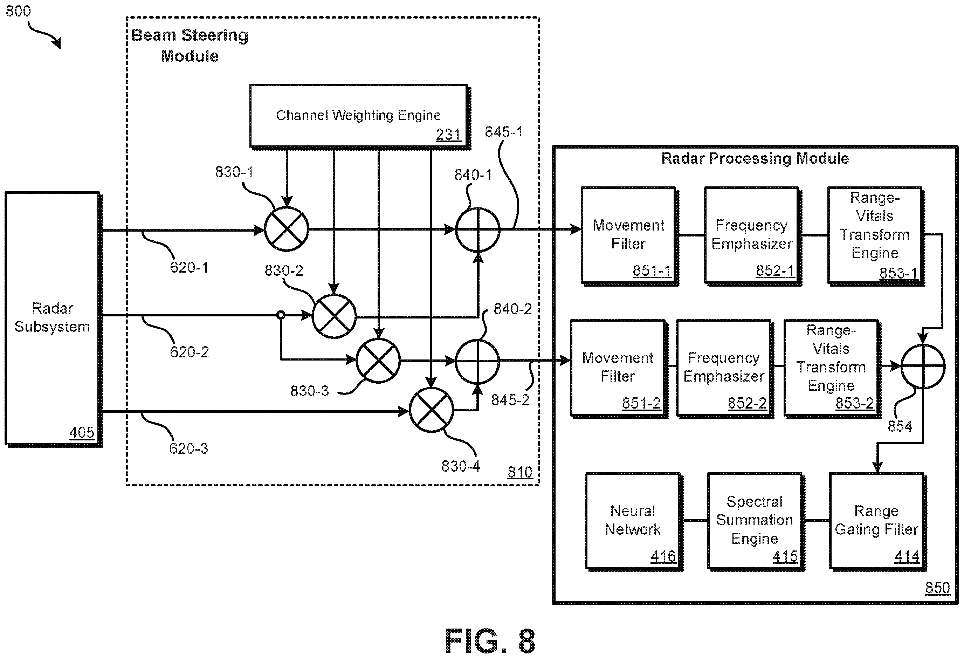

Each of beam steering modules 530 receive the same waveform data from radar subsystem 405. Therefore, for a particular received reflected chirp, each beam steering module of beam steering modules 530 targets a different spatial zone. By each of beam steering modules 530 operating based on the same waveform data from radar subsystem 405, the beam-steered waveform data output by beam steering modules 530 correspond to a same point in time. FIGS. 6 and 8 illustrate various embodiments of how beam steering modules 530 may be implemented.

Beam steering module 430 may have been used to determine a general direction to a user's chest. This direction can serve as a baseline for determining the direction to various spatial regions corresponding to different portions of the user's chest. Following beam steering module 430 determining a direction that yields strong measurements of the user's vital signs, the direction may be incremented in a horizontal direction by fixed or variable angles to yield each spatial zone to be monitored. Based on the channel weightings determined by channel weighting engine 431 and the desired angle that the particular spatial zone should be angled with respect to a center of the user's chest, weightings may be determined by each of beam steering modules 530. The greater the number of beam steering modules 530, the smaller the angle between spatial zones may be present.

The waveform data output by each of beam steering modules 530 has been weighted to target a particular spatial zone corresponding to different overlapping or non-overlapping regions of a user's chest. The weighted waveform data output by beam steering modules 530 is then processed using separate phase capture modules 501. Each of phase capture modules 501 functions as detailed in relation to phase capture module 500A of FIG. 5A. Since each phase capture module of phase capture modules 501 is steered to a different spatial region of the user's chest, the phase, frequency, and magnitude values output by each of phase capture modules 501 corresponds to a particular region of the user's chest. For each received reflected chirp, each of phase capture modules 501 outputs indications of phase, magnitude, and frequency to a corresponding radar phase processing module of radar phase processing modules 510.

Each of radar phase processing modules 510 may include multiple components. As an example, components of radar phase processing module 510-1 are indicated: phase selector 511-1 and phase unwrapper 512-1. Each of the other radar phase processing modules 510 may have similar components, but are not illustrated for simplicity.

Each of phase selectors 511, such as phase selector 511-1, may serve to select the phase component that is expected to correspond to the user's breathing in the spatial zone being monitored. Phase selector 511-1, based on the frequency spectrum data received from phase capture module 501-1, determines the greatest magnitude frequency signal present. Since the user has already been determined to be motionless (e.g., by neural network 416), no other movement is present in the environment, and filtering has already been performed to emphasize frequencies within a particular distance range that correspond to breathing, the frequency component having the greatest magnitude can be expected to be attributed to the user's breathing. The phase of this frequency component having the greatest magnitude is determined by phase selector 511-1 and other data can be discarded. Therefore, the output of phase selector 511-1 can be a phase value between $-\pi$ and $\pi$ (or 0 and $2\pi$). As waveform data corresponding to multiple chirps is processed, the phase value output by phase selector 511-1 can be expected to increase or decrease.

Phase selectors of other radar processing modules 510 function similarly. The phase value output by phase selectors of other radar processing modules 510 can be expected to correspond to the phase corresponding to the particular spatial region of the user's chest being monitored. The greater the movement of the user's chest, the greater the change that can be expected to be observed in the change in phase over time.

Each radar phase processing module of radar phase processing modules 510 can include a phase unwrapper, such as phase unwrapper 512-2. Since the user's chest may rise and fall greater than a single wavelength, phase wrapping may occur. Phase unwrappers 512 may serve to disambiguate phase changes that exceed a single wavelength. Various phase unwrapping algorithms may be used on the instantaneous phase values output for each chirp from phase selector 511-1. For instance, Itoh's sample-by-sample phase unwrapping algorithm may be used by phase unwrappers 512 to obtain phase that can exceed the range of a single wavelength represented by $-\pi$ and $\pi$ (or 0 and $2\pi$). Therefore, the output phase unwrapper 512-1 is a phase value that can be greater than a single wavelength and can account for movement of the user's chest greater than a single wavelength in distance. The phase value output by phase unwrapper 512-1 may be fed to feature bundle compiler 520.

Feature bundle compiler 520 receives unwrapped phase values for each spatial region being monitored. The number of spatial regions being monitored corresponds to the number of dimensions of phase data received by feature bundle compiler 520. For each dimension, the unwrapped phase values are buffered for an amount of time to create a signal indicative of phase over time (e.g., five seconds, ten seconds, etc.). Therefore, in the illustrated example, phase values for four regions are received for feature bundle compiler 520, but for other embodiments, fewer or greater numbers of dimensions may be received by feature bundle compiler 520.

Feature bundle compiler 520 can determine the relative phase offset between different spatial regions. On each dimension of phase data received, feature bundle compiler 520 can perform a FFT. The output of each FFT can be a frequency, a magnitude, and a phase. For each dimension, feature bundle compiler 520 creates a phasor indicative of a magnitude and phase. The phasor created for each dimension by feature bundle compiler 520 is fed to machine learning model 540 to serve as the features to be used to perform the classification by machine learning model 540.

Machine learning model 540 can receive the phasor for each dimension created by feature bundle compiler 520. Machine learning model 540 can be a neural network, such as a feedforward neural network classifier, which may have three layers. Machine learning model 540 can receive a bundle of phasors, with each phasor indicating a magnitude and a phase. At a high level, machine learning model 540 may identify situations where a bundle includes phasors that each have a significant magnitude but have significantly different phase. For instance, two vectors (which each correspond to different spatial zones of the user's chest) may have a significant magnitude and have opposite or nearly opposite phase. This situation can be indicative of paradoxical breathing.

To train machine learning model 540, a set of truth-tagged bundles of phasors may be created where the bundles are created for users that have a known breathing condition, such as paradoxical breathing, and for persons without an abnormal breathing condition. Based on such training data, the neural network, or other form of machine learning mode, can be trained to perform a classification based on such truth-tagged training data. In some embodiments, more than one type of machine learning module is present to allow for monitoring for multiple types of breathing abnormalities.

The classifications output by machine learning model 540 can be provided to breathing data output engine 319, stored using data storage 318, and/or stored by cloud-based server system 370. If an abnormal breathing condition classification is output by machine learning model 540, such data may be stored. When a user is determined to be awake, such as in the morning, information may be presented indicating that the user should be checked by a medical professional for an abnormal breathing condition. In some embodiments, breathing data output engine 319 indicates that a particular type of abnormal breathing condition appears to be present, such as paradoxical breathing. In some embodiment, breathing data output engine 319 uses synthesized speech to indicate the detected abnormal breathing condition. Other data may also be provided to the user, such as an indication of a duration of time that the abnormal breathing condition was detected, the severity of the abnormal breathing condition, how many days the abnormal breathing condition has been detected, etc.

FIG. 6 illustrates an embodiment 600 of beam steering module 610 for targeting the direction in which sleep tracking and/or breathing monitoring is performed. Beam steering module 610 can represent an embodiment of beam steering module 430 or beam steering modules 530. Generally, beam steering module 610 may apply a weight to each radar data stream received from radar subsystem 405, sum the weighted inputs, and output the combined weighted radar data stream to radar processing module 410. The weights applied may introduce a delay to the input of a particular antenna, which can be realized by the weight being a complex value. By a delay being introduced to one or more of the radar data streams received from the antennas, the antenna receive beam can be effectively steered.

In embodiment 600, three digital radar data streams 620 (620-1, 620-2, 620-3) are received from radar subsystem 205 with each digital radar data stream corresponding to a separate antenna. Therefore, in this embodiment, three antennas are present as part of radar subsystem 205. In other embodiments, radar subsystem 205 may have fewer (e.g., 2) or greater numbers (e.g., 4, 5, 6, 7, or more) of antennas, each with a corresponding raw radar data stream output in digital form to beam steering module 610.

Mixers 630 and combiner 640 can represent beam steering system 432. Each of radar data streams 620 may be input to a separate mixer of mixers 630. Mixers 630 may be digitally implemented and may therefore represent software processes. Mixer 630-1 mixes radar data stream 620-1 with a weight, represented by a complex value, output by channel weighting engine 431. Mixer 630-2 mixes radar data stream 620-2 with a weight (which may be the same or differ from the weight applied at mixer 630-1), output by channel weighting engine 431. Mixer 630-3 mixes radar data stream 620-3 with a weight (which may be the same or different from each of the weights applied at mixers 630-1 and 630-2), output by channel weighting engine 431.

Channel weighting engine 431, which can represent a software process, may perform a training process to determine the values (e.g., complex values) representative of the weights that should be output to each of mixers 630. In other embodiments, channel weighting engine 431 may be performed by separate specialized hardware or hardware that is incorporated as part of radar subsystem 405. The digital signals representing the weights output by channel weighting engine 431 may effectively apply a greater or smaller delay to each of radar data streams 620. The weights applied via mixers 630 may be normalized to 1. Therefore, the sum of the three weights applied in embodiment 600 may sum to 1.

Beam steering system 432 and beam steering module 610 can be used to implement weighted delay and sum (WDAS) beam steering via mixers 630. Equation 5 details how WDAS can be implemented:

$$\hat{x} = \sum_{i=1}^{M} w_i * x_i = \sum_{i=1}^{M} \left( a_i * e_i^{-j\pi\theta_i} \right) * x_i. \qquad \textbf{Eq. 5}$$

In Equation 5, $w_i$ represents the channel weight, which can be a complex value to introduce phase delay; $x_i$ represents the incoming digital radar data (e.g., a FMCW radar chirp) from radar subsystem 405; $a_i$ represents the complex-valued weights that are responsible for phase-delaying different receive antenna signals with different magnitudes. The weightings output by channel weighting engine 431 may be determined by performing a least-squares optimization process. The least squares optimization process may be performed according to Equation 6.

$$minimize \left\| y - Xw \right\|^2 \qquad \textbf{Eq. 6}$$

In Equation 6, $y$ represents vectorized data generated using the target beam. X represents the radar data stream data received from radar subsystem 405; w represents the weights that are to be learned by channel weighting engine 431. As part of a training process to determine the most effective weights to target the user, various weights may be tested (e.g., in a pattern, randomly) in an attempt to obtain a minimized output of Equation 6. For example, if enough randomized weights are tested, it can be expected that the minimized output value can be obtained within an amount of error. By minimizing the output value according to the least-squares optimization process, the weights corresponding to the beam direction that most closely target where the user is located within the bed may be obtained. These weights may then be used for future monitoring of the user. Periodically or occasionally, retraining may be performed to compensate for the user moving within the bed and/or the orientation and/or location of the sleep detection device being changed.

Prior to use, weights may be determined offline to compensate for a known tilt of the radar subsystem, such as indicated in FIG. 9 and indicated by directions 950 and 952. When a user is present, the optimal direction is determined for the user, such as by sweeping or randomly selecting weights. When a user is not present, one or more directions to stationary objects that produce significant reflections can be determined such that these one or more directions can be avoided when targeting a user.

It should be understood that a learning process other than a least squares optimization process may be performed by channel weighting engine 431. For instance, in some embodiments, a user may assist in the training process by providing an input indicating a direction from the contactless sleep tracking device to where the user sleeps. In other embodiments, a different form of automated learning process may be performed to target the beam at the user.

Channel weighting engine 431 may be triggered to determine weights on system 400A being booted or turned on. If motion is detected by system 400A, such as via an on-board accelerometer, channel weights may be recalculated.

A summation of the weighted radar data streams 635 (e.g., 635-1, 635-2, and 635-3), as output by mixers 630, may be received by combiner 640. Combiner 640 may output a single summed output 645 to radar processing module 410. By at least one weight (that causes a delay) applied by mixers 630 differing from the other weights applied by mixers 630, the beam is effectively steered in a direction, which may have a vertical and/or horizontal component. Processing by radar processing module 410 may be performed as detailed in relation to FIG. 4A.

FIG. 7 illustrates an embodiment of a possible antenna layout of radar subsystem 700. Radar subsystem 700 may represent an embodiment of the integrated circuit that functions as radar subsystem 405. The entire IC may have dimensions of 6.5 mm (length 705) by 5 mm (width 704). In other embodiments, the entire IC has a length 705 by width 704 of between 7 mm by 7 mm and 4 mm by 4 mm. The illustrated embodiment of radar subsystem 405 has three receive antennas and one transmit antenna, but other embodiments may have a greater or fewer number of antennas.

Radar subsystem 700 may have receive antennas 710-1, 710-2, and 710-3 distributed in an "L" pattern. That is, antennas 710-1 and 710-2 may be aligned on axis 701 and antennas 710-2 and 710-3 may be aligned on axis 702 which is perpendicular to axis 701, as illustrated in FIG. 7. The center of antenna 710-2 may be located 2.5 mm or less from the center of antenna 710-1. The center of antenna 710-2 may be located 2.5 mm or less from the center of antenna 710-3.

Transmit antenna 710-4 may be arranged separately from the L-shaped pattern of the receive antennas 710-1, 710-2, and 710-3. That is, in some embodiments, a center of transmit antenna 710-4 is not located on an axis with antenna 710-3 that is parallel to axis 701. In some embodiments, transmit antenna 710-4 is on axis 703 with the center of antenna 710-1, with axis 703 being parallel to axis 702.

Each of antennas 710 may be hollow rectangular dielectric resonance antennas (DRAs). Each of antennas 710 may have a same set of dimensions. Alternatively, each of receive antennas 710-1, 710-2, and 710-3 may have the same dimensions and transmit antenna 710-4 may vary in dimensions from the receive antennas. In some embodiments, transmit antenna 710-4 has a larger width, such as 0.2 mm larger, than receive antennas 710-1, 710-2, and 710-3 but the same length.

In such an arrangement, the phase delay introduced by the applied weights between the radar data stream of antenna 710-1 and the data stream of antenna 710-2 may affect the vertical direction of the receive beam and the phase delay introduced by weights between the radar data stream of antenna 710-2 and data stream of antenna 710-3 may affect the horizontal direction of the receive beam (assuming the radar subsystem integrated circuit is present within the contactless sleep tracking device in approximately the same orientation).

In some embodiments, separate antennas are used for transmitting and receiving. For example, antenna 710-4 may be used exclusively for transmitting, while antennas 710-1, 710-2, and 710-3 are used exclusively for receiving.

Using a radar subsystem in which all the antennas are located on a single, relatively compact integrated circuit chip, as described, has been found to achieve a good balance of cost savings, a reasonable ability to perform receive-side beam steering, and a sufficiently wide antenna pattern in the horizontal plane that is able to encompass common bed sizes (e.g., queen, king, full, twin). At the same time, a device incorporating such a radar subsystem allows it to be placed sufficiently close to a bed (e.g., within 1 m) that it can also function as a personal assistant, including alarm clock functionality (which can replace an alarm clock), home control hub, and/or entertainment touchscreen device.

While the beam steering module of embodiment 600 does not factor in the arrangement of antennas 710 with respect to each other, embodiment 800 accommodates the topology of the antenna arrangement of radar subsystem 700. In other embodiments, antennas 710 may be arranged in a pattern other than an "L."

FIG. 8 illustrates an embodiment 800 of a beam steering module for targeting the direction in which sleep tracking and/or health monitoring is performed. In embodiment 800, the antenna arrangement (i.e., antenna topology) of radar subsystem 405 is taken into account. By taking the antenna topology into account, more accurate beam steering may be performed, which can result in more accurate tracking of a user while sleeping in the user's bed. Antenna 710-1 corresponds to radar data stream 620-1, antenna 710-2 corresponds to radar data stream 620-2, and antenna 710-3 corresponds to radar data stream 620-3. That is, a phase delay added between the data streams of antenna 710-2 of radar subsystem 405 and antenna 710-3 is used for horizontal beam targeting and a phase delay added between the data streams of antenna 710-2 and antenna 710-1 is used for vertical beam targeting. Depending on whether the data stream of antenna 710-2 is used for vertical or horizontal beam targeting, a different weight may be applied using separate digitally implemented mixers.

As in embodiment 600, in embodiment 800, separate digital radar data streams 620 are received from each antenna of radar subsystem 405. Mixers 830 and combiners 840 can represent beam steering system 432 of FIG. 4A. In embodiment 800, beam steering module 810 has four mixers 830 (830-1, 830-2, 830-3, and 830-4). Similar to embodiment 600, a value (e.g., complex value) output by channel weighting engine 431 may be mixed with each radar data stream of radar data streams 620. However, different weights may be mixed with radar data stream 620-2, with two weighted outputs created, to be used separately for horizontal and vertical beam targeting. Radar data stream 620-1 may have a weight applied via mixer 830-1 and may be combined via combiner 840-1 with radar data stream 620-2 (which had a weight applied via mixer 830-2). The weights applied at mixers 830-1 and 830-2 may sum to a normalized value of 1. Radar data stream 620-3 may have a weight applied via mixer 830-4 and may be combined via combiner 840-2 with radar data stream 620-2 which had a weight applied via mixer 830-3. The weights applied at mixers 830-3 and 830-4 may sum to a normalized value of 1.

Channel weighting engine 431 may be implemented similarly to as implemented in embodiment 600. Channel weighting engine 431 may perform a least-squares optimization process or some other optimization process to determine the optimal or near optimal direction of the receive beam. Channel weighting engine 431 may generate four outputs to be used for weighting in embodiment 800 rather than three outputs as in embodiment 600. Therefore, if a pattern or random set of values output for the weights is used as part of the least-squares optimization process, a greater number of sets of output values in embodiment 800 may be tested to obtain the optimized set of output values used to set the weights as compared to embodiment 600.

Two outputs 845, output 845-1 and output 845-2, may be output to radar processing module 850. For a sleep analysis, separate processing may then be performed by radar processing module 850 for output 845-1 and output 845-2. At a high level, processing by radar processing module 850 may be performed on each of outputs 845 until direction is no longer used during processing. In embodiment 800, separate instances of movement filters, frequency emphasizers, and range-vital transform engines may be applied to each of outputs 845, then the results may be averaged or summed together. More specifically, output 845-1 may be output to movement filter 851-1, followed by frequency emphasizer 852-1, followed by range-vitals transform engine 853-1. Output 845-2 may be output to movement filter 851-2, followed by frequency emphasizer 852-2, followed by range-vitals transform engine 853-2. Movement filters 851, frequency emphasizers 852, and range-vital transform engines 853 may function as detailed in relation to movement filter 411, frequency emphasizer 412, and range-vitals transform engine 413 of FIG. 4A. The output of range-vitals transform engine 853-1 and range-vitals transform engine 853-2 may be summed or combined using combiner 854. The output of combiner 854, which can represent an average of the outputs of range-vitals transform engines 853, may be processed by range gating filter 414 and later components as detailed in relation to FIG. 4A.

In a first set of embodiments, for analyzing a user's breathing using embodiment 800, beam steering in the vertical direction is not performed. In such embodiments, only beam steering using raw waveform data from horizontally-distributed antennas may be used. For example, referring to FIG. 7, only the raw waveform data obtained from antennas 710-2 and 710-3 may be used for horizontal beam steering. The output 845-2 from beam steering module 810 may then be input to a corresponding phase capture module of phase capture modules 501. In such embodiments, the components related to vertical beam forming are not be implemented or could be used for beam targeting for sleep tracking purposes.

In a second set of embodiments, for analyzing a user's breathing using beam steering performed using embodiment 800, both outputs 845-1 and 845-2 from beam steering module 810 may be used. In such embodiments being implemented as part of system 500B, each of these outputs may be processed using separate phase capture modules of phase capture modules 501 and separate radar phase processing modules of radar phase processing modules 510, thereby creating a pair of unwrapped phase values. Each pair's two phase values may then be averaged together and passed to feature bundle compiler 520. Therefore, to perform both the vertical and horizontal beam forming of embodiment 800, it may be necessary to implement double the number of phase capture modules and radar phase processing modules (in addition to performing averaging) in order to monitor a same number of spatial regions as when only horizontal beam steering is performed.

For sleep monitoring, Since radar processing module 850 and beam steering module 810 may be performed as a software process executed by a general-purpose processor (or multiple processors), implementing more complex mixing, weights, and multiple instances of movement filters 851, frequency emphasizer 852, and range-vitals transform engine 853 may require that enough processing capability be available. Therefore, assuming such processing power is available, no hardware changes to the contactless sleep tracking device may be needed to implement embodiment 800 instead of embodiment 600. In some embodiments, embodiment 600 may be implemented and if sleep tracking results are inaccurate, embodiment 800 may be implemented (or the reverse). Advantageously, in some embodiments in which the contactless health monitoring device 300 comprises a smart-home management device (e.g., a Nest Home Hub) into which the radar functionalities are integrated, the smart-home management device being a network-connected combination of smart speaker, home assistant, and touchscreen-based control and/or entertainment hub, improvements to parameter computation methods and even entire radar processing algorithms can be achieved by in-the-field software updates pushed out as needed by a central cloud server via the Internet.

While the receive-side beam steering aspects of embodiments 600 and 800 of FIGS. 6 and 8, respectively, are implemented in the digital domain, the functionality of beam steering modules 610 and 810 may be implemented in the analog domain using analog components. If such beam steering is performed in the analog domain, conversion to the digital domain may be performed following such analog beam steering being performed such that digital data is provided to radar processing module 410 or 850.

**FIG. 9** illustrates an embodiment of a contactless sleep tracking device 900 ("device 900"). Alternatively, device 900 can be referred to as a contactless breathing tracking device. Device 900 may have a front surface that includes a front transparent screen 940 such that a display is visible. Such a display may be a touchscreen. Surrounding front transparent screen 940 may be an optically opaque region, referred to as bezel 930, through which radar subsystem 405 may have a field-of-view of the environment in front of device 900.

For purposes of the immediate following description, the terms vertical and horizontal describe directions relative to the bedroom in general, with vertical referring to a direction perpendicular to the floor and horizontal referring to a direction parallel to the floor. Since the radar subsystem, which may be an Infineon® BGT60 radar chip, is roughly planar and is installed generally parallel to bezel 930 for spatial compactness of the device as a whole, and since the antennas within the radar chip lie in the plane of the chip, then, without beam targeting, a receive beam of radar subsystem 320 may be pointed in direction 950 that is generally normal to bezel 930. Due to a departure tilt of bezel 930 away from a purely vertical direction, which is provided in some embodiments to be about 25 degrees in order to facilitate easy user interaction with a touchscreen functionality of the transparent screen 940, direction 950 may point upwards from horizontal by departure angle 951. Assuming device 900 will typically be installed on a bedside platform (e.g., nightstand) that is roughly the same height as the top of a mattress on which a user will sleep, it may be beneficial for the receive beam of radar subsystem 320 to be targeted in horizontal direction 952 or an approximately horizontal (e.g., between -5° and 5° from horizontal) direction. Therefore, vertical beam targeting can be used to compensate for departure angle 951 of the portion of device 900 in which radar subsystem 320 is present.

FIG. 10 illustrates an exploded view of an embodiment of device 900. Device 900 can include: display assembly 1001; display housing 1002; main circuit board 1003; neck assembly 1004; speaker assembly 1005; base plate 1006; mesh network communication interface 1007; top daughterboard 1008; button assembly 1009; radar assembly 1010; microphone assembly 1011; rocker switch bracket 1012; rocker switch board 1013; rocker switch button 1014; Wi-Fi assembly 1015; power board 1016; and power bracket assembly 1017. Device 900 can represent an embodiment of how device 301 may be implemented.

Display assembly 1001, display housing 1002, neck assembly 1004, and base plate 1006 may collectively form a housing that houses all of the remaining components of device 900. Display assembly 1001 may include an electronic display, which can be a touchscreen, that presents information to a user. Display assembly 1001 may, therefore, include a display screen, which can include a metallic plate of the display that can serve as a grounding plane. Display assembly 1001 may include transparent portions away from the metallic plate that allow various sensors a field of view in the general direction in which display assembly 1001 is facing. Display assembly 1001 may include an outer surface made of glass or transparent plastic that serves as part of the housing of device 900.

Display housing 1002 may be a plastic or other rigid or semi-rigid material that serves as a housing for display assembly 1001. Various components, such as main circuit board 1003; mesh network communication interface 1007; top daughterboard 1008; button assembly 1009; radar assembly 1010; and microphone assembly 1011 may be mounted on display housing 1002. Mesh network communication interface 1007; top daughterboard 1008; radar assembly 1010; and microphone assembly 1011 may be connected to main circuit board 1003, using flat wire assemblies. Display housing may be attached with display assembly 1001, using an adhesive.

Mesh network communication interface 1007 may include one or more antennas and may enable communication with a mesh network, such as a Thread-based mesh network. Wi-Fi assembly 1015 may be located a distance from mesh network communication interface 1007 to decrease the possibility of interference. Wi-Fi assembly 1015 may enable communication with a Wi-Fi based network.

Radar assembly 1010, which can include radar subsystem 320 or radar subsystem 405, may be positioned such that its RF emitter and RF receiver are away from the metallic plate of display assembly 1001 and are located a significant distance from mesh network communication interface 1007 and Wi-Fi assembly 1015. These three components may be arranged in approximately a triangle to increase the distance between the components and decrease interference. For instance, in device 900, a distance of at least 74 mm between Wi-Fi assembly 1015 and radar assembly 1010 may be maintained. A distance of at least 108 mm between mesh network communication interface 1007 and radar assembly 1010 may be maintained. Additionally, distance between radar assembly 1010 and speaker 1018 may be desired to minimize the effect of vibrations on radar assembly 1010 that may be generated by speaker 1018. For instance, for device 900, a distance of at least 79 mm between radar assembly 1010 and speaker 1018 may be maintained. Additionally, distance between the microphones and radar assembly 1010 may be desired to minimize any possible interference from the microphones on received radar signals. Top daughterboard 1008 may include multiple microphones. For instance, at least 12 mm may be maintained between a closest microphone of top daughterboard 1008 and radar assembly 1010.

Other components may also be present. A third microphone assembly may be present, microphone assembly 1011, which may be rear-facing. Microphone assembly 1011 may function in concert with the microphones of top daughterboard 1008 to isolate spoken commands from background noise. Power board 1016 may convert power received from an AC power source to DC to power the components of device 900. Power board 1016 may be mounted within device 900 using power bracket assembly 1017. Rocker switch bracket 1012, rocker switch board 1013, and rocker switch button 1014 may be collectively used to receive user input, such as up/down input. Such input may be used, for example, to adjust a volume of sound output through speaker 1018. As another user input, button assembly 1009 may include a toggle button that a user can actuate. Such a user input may be used to activate and deactivate all microphones, such as for when the user desires privacy and/or does not want device 900 to respond to voice commands.

FIG. 11 illustrates an embodiment of a state machine 1100 for determining when a person is sleeping. Based upon data output by radar processing module 312, sleep state detection engine 314 may determine whether a person is sleeping using state machine 1100. It should be understood that in some embodiments, sleep state detection engine 314 is incorporated as part of the functionality of radar processing module 312 and does not exist as a separate module. State machine 1100 may include five possible sleep states: entering bed state 1101; not in bed state 1102; motion in bed state 1103; no motion in bed state 1105; and exiting bed state 1104.

If no motion-indicative waveform data is present, this may be indicative that the user is not in bed. A user who is in bed can be expected to always be moving in at least small amounts due to their vital signs. Therefore, if zero movement is observed, the user may be judged to be in state 1101. Following state 1101 being determined, the next possible state that may be determined is state 1102. In state 1102, the monitored user is entering bed. Significant user motion may be sensed, such as according to Table 2. This may be indicative of a user entering bed and may cause the state to transition from state 1101 to state 1102.

From state 1102, motion may continue to be detected in bed, such as due to the user rolling around, getting positioned, moving pillows, sheets, and/or blankets, reading a book, etc. State 1102 may transition to state 1103 while such motion continues to be detected. Alternatively, if motion is detected, then zero motion is detected, this may be indicative that state 1105 has been entered by the monitored user exiting bed. If this condition occurs, state 1102 may transition to state 1105, then back to state 1101. Generally, state 1104 may be interpreted as the user being asleep and state 1103 may be interpreted as the user being awake. In some embodiments, more than a threshold amount of time (or some other form of determination that uses a form of threshold criterion at least partially based on time) in state 1104 is necessary to classify the user as asleep and more than a threshold amount of time (or some other form of determination that uses a form of threshold criterion at least partially based on time) in state 1103 is necessary to classify the user as awake. For instance, movement in bed of less than five seconds may be interpreted as the user moving while still asleep if the user was previously determined to be asleep. Therefore, if a user transitions to state 1103 from state 1104, experiences some number of movement events, then returns to state 1104 within less than a duration of time, the user may be identified as having experienced a "sleep arousal" in which the user's sleep is disturbed, but the user has not been awoken. Such sleep arousals may be tracked together with or separate data may be maintained from episodes where the user is judged to have fully awoken.

From state 1103, the monitored user may be determined to be exiting bed at state 1105 and may become motionless at state 1104. To be "motionless" at state 1104 refers to no large movements being performed by the monitored user, but the user continuing to perform small motions due to vital signs. In some embodiments, only when the monitored user's state is determined to be state 1104 are vital signs treated as accurate and/or stored, recorded, or otherwise used to measure the user's vital signs. Data collected during state 1103 and state 1104 may be used to determine the monitored user's general sleep patterns (e.g., how much time tossing and turning, how much quality sleep, when deep sleep occurred, when REM sleep occurred, etc.). After a user enters state 1104 for a predefined period of time, the user may be assumed to be asleep until the user exits state 1104. When a user initially transitions to state 1104, the user may be required to stay in state 1104 for some amount of time, such as two to five minutes, to be considered asleep. If a user is in state 1103 for at least a defined period of time, the user may be identified as awake. However, if the user enters state 1103 from state 1104 for less than the defined period of time, and returns to state 1104, the user may be identified as just moving within their sleep and has been continuously asleep.

The systems, devices, and state machines of FIGS. 3-11 can be used to perform various methods. **FIG. 12** illustrates an embodiment of a method for performing contactless monitoring of a user's breathing to determine if an abnormal breathing condition is present. Method 1200 may be performed using contactless health monitoring device 301. More specifically, method 1200 may be performed using system 500B of FIG. 5B.

At block 1210, radio waves are emitted. The radio waves emitted can be continuous-wave radar, such as FMCW. The FMCW radar can transmit radio waves as detailed in relation to FIG. 4B. The radio waves may be emitted by RF emitter 406 of radar subsystem 405. At block 1220, reflections of the radio waves may be received, such as by multiple antennas of RF receiver 440 of radar subsystem 405. The reflections received at block 1220 may be reflected off of moving objects (e.g., a person sleeping in bed, a person moving in bed) and stationary objects.

At block 1230, a determination is made as to whether a user is eligible for a breathing analysis. The determination of block 1230 can be performed based on whether a machine learning classification determines that the user is present in bed and is motionless (besides vital signs). The machine learning classification may be performed by neural network 416. Therefore, system 400A may be active, functioning as detailed in relation to FIG. 4A, determining a state of the user in accordance with state machine 1100, and, possibly, monitoring vital signs, such as breathing and heartrate of the user. If block 1230 is determined in the affirmative, method 1200 can proceed to block 1230 and system 500B may be activated. If block 1230 is determined in the negative, method 1200 can return to block 1210 and continue monitoring for whether the user is eligible for a breathing analysis to be performed.

At block 1235, beamforming is performed for an n number of spatial zones corresponding to the user's chest for a received reflected radar chirp. A general direction to the user's chest may first be determined by a beam steering module, such as beam steering module 430, by determining a direction in which vital signs of a user are detected with a greatest magnitude as detailed in relation to Equation 6. Based upon the direction determined, n number of spatial zones, each involving different digital receive-side beam-steering, may be performed. Each targeted spatial region may be spaced by an angle based upon the direction determined to be the direction in which vital signs of the user were detected with the greatest magnitude. The spatial zones may partially overlap.

At block 1240, an amount of breathing displacement for each of the $n$th number of spatial zones may be determined. The amount of movement by the user's chest in the spatial zone may be most accurately determined using phase. For each spatial zone, a phase capture module may be executed that outputs an indication of phase for a frequency associated with the user's breathing. This phase value may be determined and modified as detailed in relation to phase capture modules 501 and radar phase processing modules 510. Therefore, the output of block 1240 may be indicating of the instantaneous position of the user's chest. This instantaneous position may be represented by an unwrapped phase value.

In some embodiments, processing for each of the n spatial zones is performed in parallel. In other embodiments, processing is performed serially. If performed serially, after block 1240 has been performed for a first spatial zone, a determination is made at block 1250 whether blocks 1235 and 1240 need to be processed for a next spatial zone. If so, method 1200 returns to block 1235 to perform receive-side beam forming (corresponding to the same received reflected radar chirp) corresponding to another spatial zone being monitored. The number of spatial zones varies by embodiment. Embodiments can include 2, 3, 4, 5, 6, 7, 8, 9, 10, or more spatial zones.

Following all spatial zones being analyzed, either in parallel or in serial, for a given received reflected radar chirp, an n-dimensional feature vector can be created at block 1260. Block 1260 may be performed by feature bundle compiler 520 as detailed in relation to FIG. 5B. Creation of the n-dimensional feature vector can include the phase values for each spatial zone being buffered for a period of time. For each buffered set of phase values, a Fourier transform (e.g., FFT) may be performed to determine a frequency magnitude and phase value. The phase and magnitude value can be used as a phasor, or feature vector, that is input to block 1270. At block 1260, *n* number of feature vectors are created such that a feature vector corresponds to each of the n spatial zones being monitored.

At block 1270, the *n* feature vectors are analyzed, such as using a trained machine learning model. The trained machine learning model may be a neural network. The *n* feature vectors function as the features used by the machine learning model to perform a classification. The machine learning model may have been trained using a set of truth-tagged bundles of feature vectors that correspond to persons with a particular breathing condition (e.g., paradoxical breathing) and persons with healthy breathing. The machine learning model may be trained to identify users with a particular abnormal breathing condition. In some embodiments, block 1270 may be performed multiple times using different machine learning models to identify different breathing-related health conditions. The output of block 1270 may be one or more classifications along with a timestamp of the data from which the classification was made.

At block 1280, the classification and timestamp data obtained from block 1270 may be stored, analyzed, and/or output. In some embodiments, the classification data is stored for a period of time, such as a night (e.g., from when the user went to sleep until the user woke up in the morning, possibly with the user waking up one or more times during the night). The data may be compiled, stored, and/or output. In order for a user to be informed that he may have an abnormal sleeping condition, a classification that the user has the abnormal sleeping condition might need to be output for at least a threshold period of time or satisfy some other time-related criterion. For example, if a classification of paradoxical breathing is output for only several seconds during the course of an entire night, the user might not be informed about the condition. However, if the user is classified as having paradoxical breathing for more than a minute in a night, the user may receive a notification that they experienced paradoxical breathing.

A user may be provided with a nightly report that indicates whether the user experienced any abnormal breathing conditions during the night. The nightly report may be integrated with a nightly report that indicates vital sign related data, such as related to heartrate and/or breathing rate. If an abnormal breathing condition is detected, an amount of time that the user experienced the breathing condition may also be output. A definition of the breathing condition may also be output. The nightly report may be presented using the display screen of the contactless device and/or may be output using synthesized speech. In some embodiments, nightly report data is accessible by the user logging into an account stored by a cloud-based server system (such as via an application installed on the user's smartphone or tablet computer).

Additionally or alternatively, a long-term report may be output periodically or occasionally to the user (or such a report may be accessible upon request from the user). The long-term report, which may cover a time period longer than a single night, such as multiple nights, a week, two weeks, a month, six months, a year, etc., can indicate one or more trends relating to abnormal breathing conditions. For instance, a long-term report could indicate a trend of whether over time the user is experiencing a breathing abnormality for a longer or shorter period of time per night. Evidence that the duration of the breathing abnormality is increasing per night may help convince the user to visit a healthcare professional for a formal diagnosis and treatment plan.

In some embodiments, the device performing method 1200 can respond to spoken commands. A user may speak a command that requests a nightly report and/or a long-term report, such as "Hi Device, how was my sleeping last night?". When the verbal command is received, the device may transmit, via a wireless network interface, the spoken command to a cloud-based server system for interpretation. In response, the cloud-based server system may indicate the data that is to be output by the device. The device may receive the data from the cloud-based server system or may receive a command from the cloud-based server system indicating locally stored data that is to be output. A graphical interface and/or synthesized speech may be output in response to the spoken command that includes the nightly report and/or long-term report information.

Various configurations may omit, substitute, or add various procedures or components as appropriate. For instance, in alternative configurations, the methods may be performed in an order different from that described, and/or various stages may be added, omitted, and/or combined. Also, features described with respect to certain configurations may be combined in various other configurations. Different aspects and elements of the configurations may be combined in a similar manner.

Specific details are given in the description to provide a thorough understanding of example configurations (including implementations). However, configurations may be practiced without these specific details. For example, well-known circuits, processes, algorithms, structures, and techniques have been shown without unnecessary detail in order to avoid obscuring the configurations.

Also, configurations may be described as a process which is depicted as a flow diagram or block diagram. Although each may describe the operations as a sequential process, many of the operations can be performed in parallel or concurrently. In addition, the order of the operations may be rearranged. A process may have additional steps not included in the figure. Furthermore, examples of the methods may be implemented by hardware, software, firmware, middleware, microcode, hardware description languages, or any combination thereof. When implemented in software, firmware, middleware, or microcode, the program code or code segments to perform the necessary tasks may be stored in a non-transitory computer-readable medium such as a storage medium. Processors may perform the described tasks.

## Claims

1. A contactless health monitoring device (210, 301), comprising:

   a housing;
   a radar sensor (320, 405) that comprises a plurality of antennas, wherein the radar sensor (320, 405) is housed by the housing; and
   a processing system (310), comprising one or more processors, in communication with the radar sensor (320, 405), housed by the housing, wherein the processing system (310) is configured to:

   receive, for each antenna of the plurality of antennas, a radar data stream (620) from the radar sensor (320, 405), thereby receiving a plurality of radar data streams (620);
   perform a beam steering process that creates a plurality of targeted spatial zone radar data streams from the received plurality of radar data streams (620);
   determine breathing displacement for a user (201) for each targeted spatial zone radar data stream of the plurality of targeted spatial zone radar data streams;
   analyze the breathing displacement for the user (201) for each targeted spatial zone radar data stream of the plurality of targeted spatial zone radar data streams; and
   output a screening result based on analyzing the breathing displacement for the user (201) in relation to time for each targeted spatial zone radar data stream of the plurality of targeted spatial zone radar data streams.

2. The contactless health monitoring device (210, 301) of claim 1,
   wherein the screening result indicates the user (201) is experiencing paradoxical breathing.

3. The contactless health monitoring device (210, 301) of claim 1,
   wherein the beam steering process comprises applying weighted delay and sum, WDAS, beam steering to create the plurality of targeted spatial zone radar data streams.

4. The contactless health monitoring device (210, 301) of claim 1,
   wherein the processing system being configured to determine the breathing displacement for the user (201) for each targeted spatial zone radar data stream of the plurality of targeted spatial zone radar data steams comprises the processing system (310) being configured to:
   determine a phase value for each targeted spatial zone radar data stream of the plurality of targeted spatial zone radar data streams.

5. The contactless health monitoring device (210, 301) of claim 1, wherein the processing system (310) being configured to analyze the breathing displacement for the user (201) for each targeted spatial zone radar data stream of the plurality of targeted spatial zone radar data streams comprises the processing system (310) being configured to: apply a pre-trained machine learning model (540).

6. The contactless health monitoring device (210, 301) of claim 5, wherein the pre-trained machine learning model (540) is a neural network.

7. The contactless health monitoring device (210, 301) of claim 1, wherein each targeted spatial zone (220) of the plurality of targeted spatial zones corresponds to a different portion of user's body.

8. The contactless health monitoring device of claim 7, wherein the plurality of targeted spatial zones comprises at least five targeted spatial zones (220).

9. The contactless health monitoring device (210, 301) of claim 1, wherein the processing system (310) is further configured to: determine, using a state machine (1100), that the user (201) is in a sleep state and not moving based on the plurality of radar data streams (620).

10. The contactless health monitoring device (210, 301) of claim 1, further comprising:

   a wireless network interface (350), housed by the housing and in communication with the processing system (310); and
   a touchscreen display (340), housed by the housing, and in communication with the processing system (310).

11. The contactless health monitoring device (210, 301) of claim 10, further comprising:

   a microphone (334) housed by the housing and in communication with the processing system (310); and
   a speaker (355) housed by the housing and in communication with the processing system (310), wherein the processing system (310) is further configured to:

      receive a spoken command regarding analyzing the breathing displacement for the user (201);
      cause the spoken request to be transmitted to a cloud-based server system (370) via the wireless network interface (360);
      receive a command via the wireless network interface (360) in response to transmitting the spoken request to the cloud-based server system (370); and
      output the screening result via the touchscreen display (340) at least partially based on the received command.

12. The contactless health monitoring device (210, 301) of claim 1, wherein

   a first of said targeted spatial zone radar streams corresponds to a chest zone of the user (201);
   a second of said targeted spatial zone radar streams corresponds to an abdomen zone of the user (201);
   said analyzing comprises detecting a phase difference between the breathing displacement of the abdomen zone and the breathing displacement of the chest zone sufficient to be indicative of a paradoxical breathing condition; and
   output screening result includes information representative of the detected paradoxical breathing condition.


**Patentansprüche**

1. Berührungslose

   Gesundheitsüberwachungsvorrichtung (210, 301),
   umfassend: ein Gehäuse;
   einen Radarsensor (320, 405), der eine Vielzahl von Antennen umfasst, wobei der Radarsensor (320, 405) in dem Gehäuse untergebracht ist; und
   ein Verarbeitungssystem (310), das einen oder mehrere Prozessoren umfasst, die mit dem Radarsensor (320, 405) in Verbindung stehen, der in dem Gehäuse untergebracht ist, wobei das Verarbeitungssystem (310) konfiguriert ist zum:

      Empfangen, für jede Antenne der Vielzahl von Antennen, eines Radardatenstroms (620) von dem Radarsensor (320, 405), wodurch eine Vielzahl von Radardatenströmen (620) empfangen wird;
      Ausführen eines Strahllenkungsprozesses, der eine Vielzahl von Zielraumzonen-Radardatenströmen aus der empfangenen Vielzahl von Radardatenströmen (620) erzeugt;

Ermitteln der Atmungsverschiebung für einen Benutzer (201) für jeden Zielraumzonen-Radardatenstrom der Vielzahl von Zielraumzonen-Radardatenströmen;

Analysieren der Atmungsverschiebung für den Benutzer (201) für jeden Zielraumzonen-Radardatenstrom der Vielzahl von Zielraumzonen-Radardatenströmen; und

Ausgeben eines Screeningergebnisses basierend auf dem Analysieren der Atmungsverschiebung für den Benutzer (201) in Bezug auf die Zeit für jeden Zielraumzonen-Radardatenstrom der Vielzahl von Zielraumzonen-Radardatenströmen.

2. Berührungslose Gesundheitsüberwachungsvorrichtung (210, 301) nach Anspruch 1, wobei das Screeningergebnis angibt, dass der Benutzer (201) paradoxe Atmung erfährt.

3. Berührungslose Gesundheitsüberwachungsvorrichtung (210, 301) nach Anspruch 1, wobei der Strahllenkungsprozess ein Anwenden einer Strahllenkung mit gewichteter Verzögerung und Summe, WDAS, umfasst, um die Vielzahl von Zielraumzonen-Radardatenströmen zu erzeugen.

4. Berührungslose Gesundheitsüberwachungsvorrichtung (210, 301) nach Anspruch 1, wobei die Konfiguration des Verarbeitungssystems zum Ermitteln der Atmungsverschiebung für den Benutzer (201) für jeden Zielraumzonen-Radardatenstrom der Vielzahl von Zielraumzonen-Radardatendatenströmen umfasst, dass das Verarbeitungssystem (310) konfiguriert ist zum:

Ermitteln eines Phasenwerts für jeden Zielraumzonen-Radardatenstrom der Vielzahl von Zielraumzonen-Radardatenströmen.

5. Berührungslose Gesundheitsüberwachungsvorrichtung (210, 301) nach Anspruch 1, wobei die Konfiguration des Verarbeitungssystems (310) zum Analysieren der Atmungsverschiebung für den Benutzer (201) für jeden Zielraumzonen-Radardatenstrom der Vielzahl von Zielraumzonen-Radardatenströmen umfasst, dass das Verarbeitungssystem (310) konfiguriert ist zum: Anwenden eines vortrainierten Maschinenlernmodells (540).

6. Berührungslose Gesundheitsüberwachungsvorrichtung (210, 301) nach Anspruch 5, wobei das vortrainierte Maschinenlernmodell (540) ein neuronales Netzwerk ist.

7. Berührungslose Gesundheitsüberwachungsvorrichtung (210, 301) nach Anspruch 1, wobei jede Zielraumzone (220) der Vielzahl von Zielraumzonen einem unterschiedlichen Teil des Körpers des Benutzers entspricht.

8. Berührungslose Gesundheitsüberwachungsvorrichtung nach Anspruch 7, wobei die Vielzahl von Zielraumzonen zumindest fünf Zielraumzonen (220) umfasst.

9. Berührungslose Gesundheitsüberwachungsvorrichtung (210, 301) nach Anspruch 1, wobei das Verarbeitungssystem (310) ferner konfiguriert ist zum: Ermitteln, unter Nutzung einer Zustandsmaschine (1100), dass sich der Benutzer (201) in einem Schlafzustand befindet und sich nicht bewegt, basierend auf der Vielzahl von Radardatenströmen (620).

10. Berührungslose Gesundheitsüberwachungsvorrichtung (210, 301) nach Anspruch 1, ferner umfassend:

eine drahtlose Netzwerkschnittstelle (350), die in dem Gehäuse untergebracht ist und mit dem Verarbeitungssystem (310) in Verbindung steht; und

eine Touchscreen-Anzeige (340), die in dem Gehäuse untergebracht ist und mit dem Verarbeitungssystem (310) in Verbindung steht.

11. Berührungslose Gesundheitsüberwachungsvorrichtung (210, 301) nach Anspruch 10, ferner umfassend:

ein Mikrofon (334), das in dem Gehäuse untergebracht ist und mit dem Verarbeitungssystem (310) in Verbindung steht; und

einen Lautsprecher (355), der in dem Gehäuse untergebracht ist und mit dem Verarbeitungssystem (310) in Verbindung steht, wobei das Verarbeitungssystem (310) ferner konfiguriert ist zum:

Empfangen eines gesprochenen Befehls in Bezug auf das Analysieren der Atmungsverschiebung für den Benutzer (201) ;

Veranlassen, dass die gesprochene Anforderung über die drahtlose Netzwerkschnittstelle (360) an ein cloudbasiertes Serversystem (370) übermittelt wird;

Empfangen eines Befehls über die drahtlose Netzwerkschnittstelle (360) in Reaktion auf das Übermitteln der gesprochenen Anforderung an das cloudbasierte Serversystem (370); und

Ausgeben des Screeningergebnisses über die Touchscreen-Anzeige (340) zumindest teilweise basierend auf dem empfangenen Befehl.

**12.** Berührungslose

Gesundheitsüberwachungsvorrichtung (210, 301) nach
Anspruch 1, wobei

ein erster der Zielraumzonen-Radarströme einer Brustzone des Benutzers (201) entspricht;

ein zweiter der Zielraumzonen-Radarströme einer Bauchzone des Benutzers (201) entspricht;

das Analysieren ein Erkennen einer Phasendifferenz zwischen der Atmungsverschiebung der Bauchzone und der Atmungsverschiebung der Brustzone umfasst, die ausreicht, um einen paradoxen Atemzustand anzuzeigen; und

das Ausgeben des Screeningergebnisses Informationen beinhaltet, die den erkannten paradoxen Atemzustand darstellen.

**Revendications**

1. Dispositif de surveillance de santé sans

contact (210, 301), comprenant : un boîtier ;

un capteur radar (320, 405) qui comprend une pluralité d'antennes, dans lequel le capteur radar (320, 405) est logé par le boîtier ; et

un système de traitement (310), comprenant un ou plusieurs processeurs, en communication avec le capteur radar (320, 405), logé par le boîtier, dans lequel le système de traitement (310) est configuré pour :

recevoir, pour chaque antenne de la pluralité d'antennes, un flux de données radar (620) à partir du capteur radar (320, 405), pour ainsi recevoir une pluralité de flux de données radar (620) ;

réaliser un processus d'orientation de faisceau qui crée une pluralité de flux de données radar de zone spatiale ciblée à partir de la pluralité reçue de flux de données radar (620) ;

déterminer un déplacement respiratoire pour un utilisateur (201) pour chaque flux de données radar de zone spatiale ciblée de la pluralité de flux de données radar de zone spatiale ciblée ;

analyser le déplacement respiratoire pour l'utilisateur (201) pour chaque flux de données radar de zone spatiale ciblée de la pluralité de flux de données radar de zone spatiale ciblée ; et

fournir en sortie un résultat de criblage sur la base de l'analyse du déplacement respiratoire pour l'utilisateur (201) en rapport avec le temps pour chaque flux de données radar de zone spatiale ciblée de la pluralité de flux de données radar de zone spatiale ciblée.

2. Dispositif de surveillance de santé sans contact (210, 301) selon la revendication 1, dans lequel le résultat de criblage indique que l'utilisateur (201) subit une respiration paradoxale.

3. Dispositif de surveillance de santé sans contact (210, 301) selon la revendication 1, dans lequel le processus d'orientation de faisceau comprend l'application d'une orientation de faisceau à retard et somme pondérés, WDAS, pour créer la pluralité de flux de données radar de zone spatiale ciblée.

4. Dispositif de surveillance de santé sans contact (210, 301) selon la revendication 1, dans lequel le fait que le système de traitement est configuré pour déterminer le déplacement respiratoire pour l'utilisateur (201) pour chaque flux de données radar de zone spatiale ciblée de la pluralité de flux de données radar de zone spatiale ciblée comprend le fait que le système de traitement (310) est configuré pour :
déterminer une valeur de phase pour chaque flux de données radar de zone spatiale ciblée de la pluralité de flux de données radar de zone spatiale ciblée.

**5.** Dispositif de surveillance de santé sans contact (210, 301) selon la revendication 1, dans lequel le fait que le système de traitement (310) est configuré pour analyser le déplacement respiratoire pour l'utilisateur (201) pour chaque flux de données radar de zone spatiale ciblée de la pluralité de flux de données radar de zone spatiale ciblée comprend le fait que le système de traitement (310) est configuré pour : appliquer un modèle d'apprentissage automatique pré-entraîné (540).

**6.** Dispositif de surveillance de santé sans contact (210, 301) selon la revendication 5, dans lequel le modèle d'apprentissage automatique pré-entraîné (540) est un réseau neuronal.

**7.** Dispositif de surveillance de santé sans contact (210, 301) selon la revendication 1, dans lequel chaque zone spatiale ciblée (220) de la pluralité de zones spatiales ciblées correspond à une partie différente du corps de l'utilisateur.

**8.** Dispositif de surveillance de santé sans contact selon la revendication 7, dans lequel la pluralité de zones spatiales ciblées comprend au moins cinq zones spatiales ciblées (220) .

**9.** Dispositif de surveillance de santé sans contact (210, 301) selon la revendication 1, dans lequel le système de traitement (310) est en outre configuré pour : déterminer, à l'aide d'un automate fini (1100), que l'utilisateur (201) est dans un état de sommeil et immobile sur la base de la pluralité de flux de données radar (620).

**10.** Dispositif de surveillance de santé sans contact (210, 301) selon la revendication 1, comprenant en outre :

une interface de réseau sans fil (350), logée par le boîtier et en communication avec le système de traitement (310) ; et
un écran tactile (340), logé par le boîtier, et en communication avec le système de traitement (310).

**11.** Dispositif de surveillance de santé sans contact (210, 301) selon la revendication 10, comprenant en outre :

un microphone (334) logé par le boîtier et en communication avec le système de traitement (310) ; et
un haut-parleur (355) logé par le boîtier et en communication avec le système de traitement (310), dans lequel le système de traitement (310) est en outre configuré pour :

recevoir un ordre oral concernant l'analyse du déplacement respiratoire pour l'utilisateur (201) ;
amener la demande vocale à être transmise à un système de serveur en nuage (370) via l'interface de réseau sans fil (360) ;
recevoir un ordre via l'interface de réseau sans fil (360) en réponse à la transmission de la demande orale au système de serveur en nuage (370) ; et
fournir en sortie le résultat de criblage par l'écran tactile (340) au moins partiellement sur la base de l'ordre reçu.

**12.** Dispositif de surveillance de santé sans

contact (210, 301) selon la revendication 1, dans lequel
un premier desdits flux radar de zone spatiale ciblée correspond à une zone thoracique de l'utilisateur (201) ;
un deuxième desdits flux radar de zone spatiale ciblée correspond à une zone abdominale de l'utilisateur (201) ;
ladite analyse comprend la détection d'une différence de phase entre le déplacement respiratoire de la zone abdominale et le déplacement respiratoire de la zone thoracique suffisante pour indiquer une affection respiratoire paradoxale ; et
le résultat de criblage fourni en sortie comprend des informations représentatives de l'affection respiratoire paradoxale détectée.

**FIG. 1**

**FIG. 2**

300

**Contactless Health Monitoring Device**

| Display | 340 |
| Wireless Network Interface | 350 |
| Speaker | 355 |

Radar Subsystem 320

330

Light Sensor 332

Microphone 334

Temperature Sensor 336

Passive Infrared Sensor 338

**Processing System**

Radar Processing Module 312

Sleep State Detection Engine 314

Breathing Monitor Engine 316

310

Data Storage 318

Breathing Data Output Engine 319

301

Network 360

370

Cloud-based Server System

# FIG. 3

400A

**Radar Subsystem**

RF Emitter
406

RF Receiver
440

Radar Processing Circuit
408

405

**Beam Steering Module**

Channel Weighting Engine
431

Beam Steering System
432

430

**Radar Processing Module**

Movement Filter
411

Frequency Emphasizer
412

Range-Vitals Transform Engine
413

Neural Network
416

Spectral Summation Engine
415

Range Gating Filter
414

410

**FIG. 4A**

FIG. 4B

EP 4 203 782 B1

500A

**Phase Capture Module**

| Movement Filter 411 | Frequency Emphasizer 412 | Phase Analysis Engine 502 |

Frequency, Phase, distance, and Magnitude Data

## FIG. 5A

**FIG. 5B**

EP 4 203 782 B1

Radar Processing Module 410

Beam Steering Module 610

Channel Weighting Engine 431

645
635-1
640
635-2
635-3
630-1
630-2
630-3
620-1
620-2
620-3

600

Radar Subsystem 405

Radar Processing Circuit 408

RF Emitter 406

RF Receiver 407

FIG. 6

**FIG. 7**

EP 4 203 782 B1

FIG. 8

**FIG. 9**

FIG. 10

1100

Not in Bed
1101

Entering Bed
1102

User Present, No
Motion in Bed
1104

Exiting Bed
1105

User Present,
Motion in Bed
1103

**FIG. 11**

1200

Emit radio waves
1210

Receive reflections of the radio waves
1220

User eligible for breathing analysis?
1230

No

Yes

Perform beamforming for an nth spatial zone of the user's chest
1235

Determine breathing displacement for the nth spatial zone
1240

Additional spatial zone to analyze?
1250

Yes

No

Create n-dimensional feature vector
1260

Analyze the breathing displacement for each targeted spatial zone
1270

Output and/or store a screening result based on analyzing the breathing displacement for each targeted spatial zone
1280

# FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2019031290 W
- US 16990705 B
- US 16990714 B
- US 16990720 B
- US 16990726 B
- US 16990746 B
- AU 2012308234 A1
- WO 2019122413 A1
- WO 2020104465 A2
- US 2011060215 A1

### Non-patent literature cited in the description

- EM techniques for the detection of breast cancer. **FAN YANG et al.** ANTENNAS AND PROPAGATION SOCIETY INTERNATIONAL SYMPOSIUM (AP-SURSI), 2010 IEEE. IEEE, 11 July 2010, 1-4